# FASCICULE DE BREVET EUROPEEN

(11) **EP 2 758 369 B1**
(45) Date de publication et mention de la délivrance du brevet: **23.03.2016**
(21) Numéro de dépôt: 12781454.9
(22) Date de dépôt: 21.09.2012
(51) Int. Cl.: C07C 279/22, A61K 31/155

(54) **NOUVEAUX COMPOSÉS MODULATEURS DE LA VOIE DE SIGNALISATION DES PROTÉINES HEDGEHOG, LEURS FORMES MARQUÉES, ET APPLICATIONS**
NEUE VERBINDUNGEN ZUR MODULATION DES HEDGEHOG-PROTEIN-SIGNALISIERUNGSPFADES, MARKIERTE FORMEN DAVON UND ANWENDUNGEN DAVON
NOVEL COMPOUNDS MODULATING THE HEDGEHOG PROTEIN SIGNALING PATHWAY, MARKED FORMS THEREOF, AND APPLICATIONS

(30) Priorité: 23.09.2011 FR 1158519
(43) Date de publication de la demande: 30.07.2014
(73) Titulaire: Centre National de la Recherche Scientifique, 75016 Paris (FR); Université de Strasbourg, 67081 Strasbourg Cedex (FR)
(72) Inventeur: RUAT, Martial, 91400 Orsay (FR); FAURE, Hélène, 91190 Gif-sur-yvette (FR); ROUDAUT, Hermine, 95670 Marly-la-ville (FR); HOCH, Lucile, 78610 Auffargis (FR); SCHOENFELDER, Angèle, 67450 Lampertheim (FR); TADDEI, Maurizio, 53035 Monteriggioni (IT); MANN, André, 67540 Ostwald (FR)
(74) Mandataire: Gevers & Orès
(86) Numéro de dépôt international: PCT/IB2012/055033
(87) Numéro de publication internationale: WO 2013/042082

(56) Documents cités:
- WO-A2-2009/130422
- ANTONIO SOLINAS ET AL: "Acylthiourea, Acylurea, and Acylguanidine Derivatives with Potent Hedgehog Inhibiting Activity", JOURNAL OF MEDICINAL CHEMISTRY, vol. 55, no. 4, 23 février 2012 (2012-02-23), pages 1559-1571, XP055024917, ISSN: 0022-2623, DOI: 10.1021/jm2013369

## Description

La présente invention est relative à de nouveaux composés de formule (I), à leur utilisation à titre de médicament, notamment pour le traitement de tumeurs associées à une hyperactivation de la voie de signalisation des protéines Hedgehog, pour le traitement de pathologies de type neuro-dégénératif, pour le traitement de maladies liées au développement cérébral (holoprosencéphalie), au contrôle de cellules souches, au traitement d'accident vasculaire cérébral et aux accidents cardiovasculaires, ainsi qu'aux maladies des oligodentrocytes et des cellules de Schwann, pour leur application *in vitro* pour moduler le renouvellement des cellules souches humaines ou animales, ainsi que pour le traitement du diabète. La présente invention concerne également des compositions pharmaceutiques comprenant, à titre de principe actif, au moins un composé de formule (I). Un procédé de radio-marquage de composés de formule (I), les composés marqués et leur utilisation comme outils de recherche font également parties de l'invention. Enfin, la présente invention concerne également une méthode de criblage et/ou d'identification de ligands des sites de liaison Smo du récepteur Smoothened, des méthodes d'identification d'agonistes et d'antagonistes du récepteur Smoothened et une méthode d'identification de cellules, telles que des cellules tumorales exprimant le récepteur Smoothened.

La molécule de signalisation Hedgehog (Hh) est une protéine auto-protéolytique sécrétée qui active la voie de signalisation des protéines Hedgehog, une voie de signalisation qui joue un rôle fondamental dans la morphogenèse de nombreux tissus, en particulier dans la formation de l'endoderme et de l'axe embryonnaire, le développement du cerveau et des follicules pileux, ainsi que dans la prolifération cellulaire, et serait impliquée dans le maintien et la réparation tissulaire chez l'adulte (Ingham et al., Genes Dev., 2001, 15, 3059-3087 ; Marti et al., Trends Neurosci., 2002, 25, 89-96 ; Weschler et al., Annu. Rev. Neurosci., 2001, 24, 385-428).

La protéine Hedgehog et la voie de transduction associée, initialement mises en évidence chez la drosophile, sont conservées chez les vertébrés et les invertébrés. Un seul homologue de Hh est présent chez la Drosophile, alors que trois homologues de Hh : Sonic (Shh), Indian (Ihh) et Desert (Dhh) sont présents chez les mammifères. Parmi ces trois homologues, Shh a été la plus étudiée du fait de son profil d'expression étendu durant le développement. Shh participe à la ventralisation du tube neural en spécifiant le phénotype précoce de plusieurs types neuronaux le long de la ligne médiane ventrale (motoneurones de la moelle épinière, neurones dopaminergiques ou cholinergiques), et en induisant la génération des précurseurs oligodendrocytaires à partir de la moelle épinière ventrale. Par ailleurs, Shh induit la survie des neurones gabaergiques et dopaminergiques, oriente le devenir des précurseurs sérotoninergiques et prévient la mort des neurones dopaminergiques provoquée par la toxine MPP. Il induit enfin la prolifération des précurseurs des cellules granulaires dans le cervelet post-natal précoce. Les autres membres de la famille Hedgehog participent, quant à eux, respectivement au développement du tissu osseux (Ihh), des testicules et des nerfs périphériques (Dhh). En outre, les résultats obtenus avec Shh s'appliquent également à Dhh et Ihh.

Shh est synthétisé sous la forme d'un précurseur qui subit une série de modifications post-traductionnelles au cours desquelles la protéine est clivée par une activité enzymatique présente dans sa partie C-terminale. Cette autoprotéolyse génère un fragment C-terminal (ShhC) et un fragment N-terminal (ShhN) qui représente le fragment actif. Lors de cette réaction, on observe également l'addition d'une molécule de cholestérol dans la partie C-terminal de ShhN, qui favorise l'ancrage de ShhN à la membrane. Enfin une acétyl-transférase permet l'ajout d'une molécule de palmitate sur un résidu cystéine près de l'extrémité N-terminale. Ces évènements produisent une protéine Shh biologiquement active. La sécrétion de la protéine est dépendante de la protéine Dispatched (Disp) dont deux isoformes Disp 1 et 2 existent chez les mammifères (Heretsch et al., 2010, Bioorg. Med. Chem. Lett. 18 : 6613-6624).

Le fragment ShhN soluble transmet son action par l'intermédiaire d'un complexe contenant deux protéines transmembranaires : Patched (Ptc) une protéine à 12 domaines transmembranaires présentant une structure de type transporteur et Smoothened (Smo) une protéine à 7 domaines transmembranaires homologue aux membres de la superfamille des récepteurs couplés aux protéines G (RCPG). Chez les mammifères, il existe une deuxième forme de Patched : Ptc2.

En absence de son ligand Shh, Ptc inhibe Smo. Une cascade intracellulaire, faisant intervenir de nombreux facteurs dont la protéine Supressor of Fused (SuFu) et la protéine PKA, est alors induite. SuFu est un régulateur négatif de la voie de signalisation Shh, il peut se lier aux trois facteurs de transcription de la famille Gli et réguler leur activation. De plus, la délétion de Sufu résulte en une activation de la voie. Les facteurs de transcription de la famille Gli sont alors phosphorylés, ubiquitinylés puis clivés sous leur forme négative (GliR) par le protéasome, GliR pénètre dans le noyau et la transcription est inactive. Lorsque Shh se lie à Ptc, l'inhibition que celui-ci exerce sur Smo est levée avec translocation nucléaire de la forme active des facteurs de transcription Gli (GliA) et activation transcriptionnelle de gènes cibles tels que ptc et gli1.

La protéine Hedgehog interacting protein (Hip) est capable de lier Shh avec une affinité comparable à celle de la protéine Ptc (Traiffort et al., J. Neurochem., 2010, 113 : 576-590). Hip est considérée comme un modulateur négatif de la voie car en liant Shh elle diminue la quantité de ligand disponible pour activer la voie de signalisation via Ptc. Cdo et Boc appartiennent à la famille des protéines de surface cellulaire possédant des motifs immunoglobulines et fibronectines de type III. Ces protéines régulent positivement la voie de signalisation Shh en facilitant la présentation du ligand Shh à Ptc, en accroissant les quantités de morphogène à proximité des cellules cibles et en affectant éventuellement l'activité des protéines Gli (Heretsch et al., 2010, Bioorg. Med. Chem. Lett 18 : 6613-24 : Scales et de Sauvage, 2009, Trends Pharmacol. Sci. 30 : 303-312).

Le rôle régulateur de la voie de signalisation des protéines Hedgehog durant le développement embryonnaire a été largement étudié : Hh a été associée aux processus de maintien et de réparation du tissu normal, à la régulation spatiotemporelle de la prolifération et de la différenciation, permettant ainsi aux tissus en développement d'atteindre leur taille correcte avec les types cellulaires appropriés et des degrés appropriés de vascularisation et d'innervation. Elle a notamment été impliquée dans le développement du système nerveux central (Dessaud et al., 2008, Development, 135 : 2489-503). Le rôle essentiel de la fonction de signalisation Hh est démontré par les conséquences dramatiques des défauts dans cette voie de signalisation chez le foetus humain, comme l'holoprosencéphalie observée avec les mutants de Shh (Traiffort et al., J. Biol. Chem., 2004, 279 : 42889-42997).

Plus récemment la voie Shh a été identifiée dans le cerveau adulte, où la forme active amino-terminale de la molécule est exprimée dans de nombreuses régions du système nerveux mature suggérant de nouveaux rôles pour cette voie. En effet, elle participe notamment à l'établissement et au maintient des niches neurogéniques et régule la prolifération de précurseurs neuraux ou gliaux dans le cerveau adulte (Traiffort et al., 2010, J. Neurochem., 113 : 576-590). La modulation de la voie de signalisation Shh représente donc un enjeu pour le développement de thérapies pour les maladies neurodégénératives. Des études ont déjà permis de mettre en évidence des effets positifs de l'activation de la voie de signalisation Shh par la protéine Shh elle-même, sur la réduction des troubles du comportement de rat atteints de la maladie de Parkinson (Tsuboi et al., 2002, Exp. Neurol. 173 : 95-104) ou sur la remyélinisation de neurones de rat atteints de sclérose en plaque (Mastronardi et al. 2004, J. Immunol. 172:6418-26). De plus, il a été montré que l'activation de la voie de signalisation Shh par un agoniste de Smo permettait une augmentation de la prolifération de précurseurs neuraux au niveau des aires de neurogenèse de souris adulte (Machold et al., 2003, Neuron., 39 : 937-950). Cependant, les agonistes de Smo restent peu nombreux et encore mal caractérisés.

Des dysfonctionnements de la voie de signalisation Shh ont également été associés à de nombreux cancers. En effet, des mutations inactivatrices de *Ptc* sont associées au Syndrome de Gorlin ou naevomatose basocellulaire, une maladie autosomale dominante caractérisée par des malformations cranofaciales et cérébrales, mais surtout par une incidence élevée de diverses tumeurs, plus particulièrement des carcinomes basocellulaires au niveau cutané et des médulloblastomes, au niveau cervelet. Des mutations des gènes *Ptc* ou *Smo* humains sont également observées dans des tumeurs primitives neuroectodermales du système nerveux central, principalement des médulloblastomes (30% des cas), mais aussi dans des formes sporadiques de carcinomes basocellulaires (respectivement 40% et 20% des cas pour *Ptc* et *Smo*)*.* En outre, des mutations de Shh sont également associées à des carcinomes basocellulaires. D'autres types de tumeurs ont également été associés à un défaut de la voie de signalisation Hedgehog , la localisation de ces tumeurs est étroitement corrélée aux sites d'expression des composantes de la voie au cours du développement embryonnaire (Scales et de Sauvage, 2009, Trends in Pharmacol. Sci., 30 : 303-312). A titre d'exemple non-limitatif on peut citer : des cancers du sein et des méningiomes associés à des mutations de *Ptc,* des glioblastomes associés à des mutations de Gli, des cancers gastro-intestinaux, notamment les cancers primaires de l'estomac et du colon, des cancers de la prostate, de la vessie, des fibromes et des dermoïdes ovariens, des rhabdomyosarcomes, des cancers du poumon à petites cellules, des carcinomes oraux à cellules squameuses.

Du fait du rôle crucial de la voie de signalisation des protéines Hedgehog dans de nombreux processus physiologiques et pathologiques, les composantes de cette voie, telles que les protéines Smoothened (Smo 1 et Smo 2), Frizzled (Fz 1 à Fz 10), Patched (Ptc 1 et Ptc 2), les protéines Dispatched (Disp 1 et Disp 2) ou bien encore la protéine Hip représentent des cibles pour la mise au point de nouvelles molécules capables de moduler (activer ou inhiber) cette voie et donc de réguler positivement ou négativement le développement [prolifération, différenciation, migration, survie (apoptose)] et/ou l'activité de cellules différenciées et de cellules souches, *in vitro* et/ou *in vivo* chez l'embryon ou chez l'adulte.

Il est démontré que de telles molécules sont utiles dans le traitement des tumeurs associées à une hyperactivation de la voie Hedgehog (Scales et de Sauvage, 2009, Trends in Pharmacol. Sci., 30 : 303-312). De telles molécules seraient donc utilisables dans le traitement de tumeurs variées telles que les tumeurs du tissu nerveux (médulloblastomes, tumeurs primitives neuroectodermiques, glioblastomes, méningiomes et oligodendrogliomes), des tumeurs cutanées (carcinomes basocellulaires, trichoépithéliomes), les tumeurs des tissus musculaires et osseux (rhabdomyosarcomes, ostéosarcomes, mélanomes) et les tumeurs d'autres tissus (rein, vessie, prostate, poumon, estomac, pancréas, sein, foie).

De telles molécules sont également utiles dans le traitement des pathologies de type neuro-dégénératif nécessitant un blocage de la voie Hedgehog (maladie de Parkinson, Chorée de Huntington, maladie d'Alzheimer, sclérose en plaques, maladie du motoneurone), et des maladies dans lesquelles le blocage de la voie de signalisation Hedgehog pourrait être bénéfique comme le diabète.

De telles molécules sont également utiles dans le traitement médical ou chirurgical (chirurgie plastique ou réparatrice, greffe de tissus ou d'organes) de nombreuses pathologies aiguës, subaiguës ou chroniques, génétiques ou acquises - impliquant un dysfonctionnement tissulaire lié à une dérégulation de la voie Hedgehog -, pour induire la formation, la régénération, la réparation et/ou l'augmentation de l'activité de tissus tels que le tissu nerveux [système nerveux central (cerveau) et périphérique (neurones sensoriels, moteurs, sympathiques)], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et les cellules du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuses) et les troubles de la spermatogenèse.

Différentes molécules, capables de moduler l'activité de la voie Hedgehog, ont été identifiées.

En premier lieu, les protéines Hedgehog et des polypeptides dérivés (fragments, variants...), notamment des agonistes et des antagonistes des protéines Hedgehog (Demande Internationale PCT WO 01/98344 au nom de BIOGEN) ; du fait de leur taille, ces protéines et les polypeptides dérivés ne peuvent pas passer la barrière hématoencéphalique et ne peuvent donc pas être administrés par voie systémique, notamment pour le traitement des tumeurs cérébrales liées à une hyperactivation de la voie de signalisation des protéines Hedgehog. En outre, de telles molécules sont peu stables, et difficiles à produire et à purifier. A l'inverse, il existe des molécules qui inhibent l'effet du ligand Shh, la robotnikinin et l'anticorps monoclonal 5E1.

La voie de signalisation Hh peut également être modulée plus en aval. L'effet inhibiteur de Ptc sur Smo peut par exemple être modulé. Il est augmenté par les statines et réduit par les oxystérols par un mécanisme qui n'est pas encore bien compris (Heretsch et al., Bioorg. Med. Chem. Lett., 2010, 18 : 6613-6624). Des produits naturels (Physalin F) ou synthétiques (GANT58, GANT61, HPI-1) sont également connus pour inhiber la liaison des facteurs de transcription Gli à l'ADN dans le noyau.

Toutefois, la plupart des recherches se sont concentrées sur la découverte de modulateurs agissant au niveau du récepteur Smoothened :
- des molécules organiques hétérocycliques inhibant ou activant (SAG et dérivés) la voie de signalisation Hh : Demande Internationale PCT WO 01/74344 au nom de CURIS ; Chen et al., PNAS, 2002, 99, 14071-14076,
- la purmorphamine une petite molécule activant la voie de signalisation Hh : Wu et al., Chemistry & Biology, 2004, 1229-1238,
- des molécules hétérocycliques azotées : Demandes Internationales PCT WO 01/19800, WO 01/26644 et WO 02/30421 au nom de CURIS ; Kamenetsky et al., J. Biol., 2002, 1, 1-19,
- des stéroïdes végétaux dérivés de *Veratrum* spp (jervine, cyclopamine et cycloposine) et de *Solanum* spp. (solanidine), substitués en position 16, 17 ou 18 par une amine ou un dérivé d'amine, et du cholestérol : Brevet américain US 6,432,970 et Demandes Internationales PCT WO 99/52534 et WO 01/27135 au nom de JOHNS HOPKINS UNIVERSITY SCHOOL OF MEDICINE; Brevet américain US 6,291,516 ; Demande Internationale PCT WO 00/41545 au nom de ONTOGENY INC. ; Demande Internationale PCT WO 02/30462 au nom de CURIS ; Taipale et al., Nature, 2000, 406, 1005-1009; Berman et al., Science, 2002, 297, 1559-1561. Toutefois, il a été démontré que des concentrations en cyclopamine supérieures à 10 µM s'avéraient être cytotoxiques pour les cellules (Borzillo et al., Curr. Top Med. Chem., 2005, 5(2), 147-157). De plus, les effets *in vivo* de la cyclopamine sur la croissance tumorale ont été remis en question car ils pourraient être liés à une activité en dehors de la tumeur elle-même (Yauch et al., 2008, Nature, 455 : 406-410). Un dérivé de la cyclopamine (IPI-926) est actuellement en phase clinique II (Mahindroo et al., J. Med. Chem., 2009, 52, 3829 ; Tremblay et al., J. Med. Chem., 2009, 52 : 14, 4400-4418),
- la mifepristone (17β-hydroxy 11β-(4-diméthylamino phényl) 17α-(prop-1-ynyl)estra-4,9-dien 3-one), également dénommée RU-486 ou RU-38486 (Brevet français FR 2 850 022 au nom du CNRS) pour laquelle une activité inhibitrice de l'activité de la voie de signalisation des protéines Hedgehog a été démontrée,
- les molécules SANT74 et le SANT75 ayant une structure analogue à celle du SAG, composé activateur synthétique de type chlorobenzothiophène (CAS N° : 364590-63-6) sont également connus pour être des inhibiteurs stables permettant de contrôler efficacement la conformation de l'activateur *Smo (*Yang et al., The Journal of Biological Chemistry, publié le 14 avril 2009).

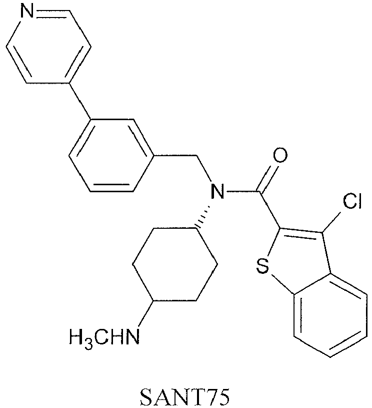

Plus récemment, d'autres composés inhibiteurs de la voie de signalisation Hedgehog ont également été décrits (Peukert et Miller-Moslin, 2010, ChemMedChem 5 : 500-512 ; Low et De Sauvage, 2010, J. Clin. Oncol. ; Ng et Curran, 2011, Nature Review Cancer) :
- Des inhibiteurs à base de bisamide (Demande Internationale PCT WO 2007/059157 au nom de GENENTECH INC. et CURIS INC.) et de pyridyle (Demande Internationale PCT WO 2006/028958 au nom de GENENTECH INC. et CURIS INC ; Brevet US 2009/0281089 au nom de GENENTECH INC.). Un des composés à base pyridyle, le GDC-0449 (phase clinique II) a montré son efficacité chez un patient atteint de métastases de médulloblastome. Toutefois, le patient a progressivement développé une résistance à la molécule. Une mutation d'un acide aspartique (D473H) de Smo est apparue. Elle perturbe la capacité du composé à se lier à Smo et à inhiber cette voie. Une mutation du même acide aminé a été identifiée chez un modèle souris atteint de médulloblastomes et traité par ce composé (Yauch et al., 2009, Science 326 : 572-574).
- Des inhibiteurs développés par la société Novartis. Par exemple, le LDE225 (phase clinique II) a été testé pour traiter des médulloblastomes chez le modèle souris et a induit une régression de ces tumeurs. Toutefois, une résistance a été observée au cours du traitement. Une étude a révélé plusieurs mécanismes de résistance dont une amplification chromosomique de Gli2 et, plus rarement, des mutations ponctuelles du récepteur Smo qui conduisent à la réactivation de la croissance tumorale. Ils ont également identifié une régulation positive de la signalisation de la phosphatidylinositol 3-kinase (PI3K) (Buonamici et al., 2010, Sci. Transl. Med., 51-70).
- Des inhibiteurs développés par Bristol-Myers Squibb Inc tels que le BMS-833923 (XL-139).
- Des inhibiteurs décrits par la société Pfizer Products, Inc. (WO 2008/075196 et US 2009/0005416). Le PF-04449913 est actuellement en phase clinique II.
- Des inhibiteurs décrits par la société MERCK (Dessole et al., 2009, Bioorg. & Med. Chem. Lett. 19 : 4191-4195).
- Les composés LEQ506 de Novartis et le TAK-441 de la société Millennium viennent également d'entrer en phase clinique I.
- Des inhibiteurs à base d'acyl-urées, -thiourées et -guanidines ont également été protégés en tant que modulateurs de la voie Hedgehog (WO 2009/130422 et WO 2011/010013 au nom du CNRS). Ces derniers ont le mérite d'être faciles à préparer en comparaison aux autres molécules existantes. Les dérivés acyl-guanidines sont, de plus, solublent dans l'eau.

Les Inventeurs se sont maintenant donnés pour but de pourvoir à de nouveaux composés modulateurs (stimulateurs ou inhibiteurs) de la voie de signalisation des protéines Hedgehog qui répondent mieux aux besoins de la pratique, notamment en ce qu'ils sont simples à synthétiser et potentiellement utilisables en thérapie humaine.

Cet objectif est atteint par les composés de formule (I) qui sont décrits ci-après et qui constituent le premier objet de l'invention, ces composés étant les plus puissants antagonistes du récepteur Smoothened identifiés à ce jour (5 à 30 fois plus puissants que les composés actuellement en phase clinique). Ces molécules présentent également une affinité 10 à 30 fois supérieure à celle des molécules GDC-0449 et LDE225, ces dernières s'étant par ailleurs heurtées à l'apparition de résistance chez certains patients (résistances en particulier liées à l'apparition de mutations sur le récepteur Smoothened). En outre, les molécules de l'invention présentent l'avantage d'être facilement préparées, généralement en trois ou quatre étapes, selon des procédés de synthèse analogues aux procédés classiques connus de l'homme de l'art, les composés acyl-urée, -thiourée et guanidine étant facilement accessibles à partir de matières premières disponibles. La fonction guanidine, en tant que base, est salifiable, ce qui a l'avantage de produire des composés ayant une bonne solubilité en milieu aqueux. L'ensemble des composés de formule (I) est obtenu de manière très commode en utilisant des réactions chimiques simples bien connues de l'homme de l'art.

La présente invention a pour objet les composés de formule (I) suivante : 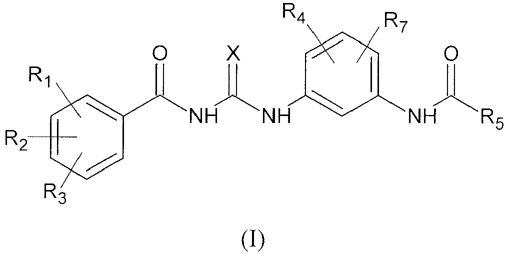 dans laquelle :
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy, alkylthio ou nitrile, lesdits groupes alkyle, perfluoroalkyle, alcoxy et alkylthio pouvant comprendre de 1 à 6 atomes de carbone,
- X représente O, S ou NH, et de préférence X est NH,
- R₄ et R₇, identiques ou différents et indépendamment l'un de l'autre, représentent un atome d'hydrogène ou d'halogène ou un groupe alkyle,
- R₅ représente un des groupes choisis parmi : 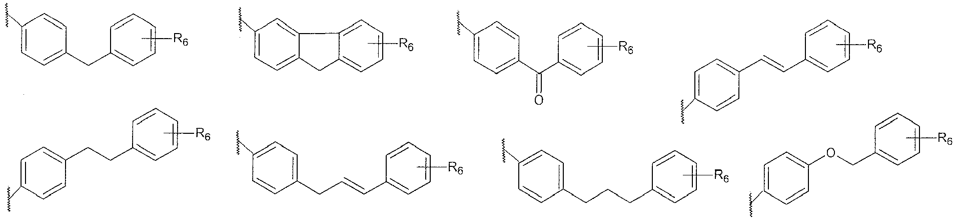 substitué par au moins un radical R₆ représentant un atome d'halogène ou un groupe alkyle, alcoxy, aminoalkyle, thioalkyle ou hydroxyle, lesdits groupes alkyle, alcoxy, aminoalkyle et thioalkyle pouvant comprendre de 1 à 6 atomes de carbone.

Au sens de la présente invention, on entend par :
- Alkyle : un groupe aliphatique hydrocarboné saturé, linéaire ou ramifié, ayant de 1 à 6 atomes de carbone, de préférence de 1 à 2 atomes de carbone. Le terme « ramifié » signifie qu'au moins un groupe alkyle inférieur tel qu'un méthyle ou un éthyle est porté par une chaîne alkyle linaire. Le terme alkyle "inférieur" désigne un alkyle ayant 1 ou 2 atomes de carbone ; le terme "alkyle supérieur" désigne un groupe alkyle linéaire ou ramifié ayant de 3 à 6 atomes de carbone. A titre de groupe alkyle, on peut mentionner par exemple les groupes méthyle, éthyle, n-propyle, i-propyle, n-butyle, t-butyle et n-pentyle.

- Atome d'halogène : désigne un atome de brome, de chlore, d'iode ou de fluore ; les désignations brome, chlore et fluore étant préférées ;
- Perfluoroalkyle : désigne un groupe alkyle tel que défini ci-dessus dans lequel tous les atomes d'hydrogène ont été remplacés par des atomes de fluore. Parmi les groupes perfluoroalkyle, les groupes trifluorométhyle et perfluoroéthyle sont préférés ;
- Alcoxy : désigne un groupe O-alkyle dans lequel le groupe alkyle peut prendre la même signification que celle indiquée ci-dessus. A titre d'exemple de groupes alcoxy, on peut notamment citer les groupes méthoxy, éthoxy, n-propoxy, iso-propoxy, n-butoxy et pentoxy ;
- Alkylthio : désigne un groupe alkyl-S dans lequel le groupe alkyle peut prendre la même signification que celle indiquée ci-dessus. A titre d'exemples de groupe alkylthio, on peut notamment citer les groupes méthylthio, éthylthio, iso-propylthio, butylthio et pentylthio ;
- Aminoalkyle : désigne un groupe alkyl-N dans lequel le groupe alkyle peut prendre la même signification que celle indiquée ci-dessus. A titre d'exemples de groupe aminoalkyl, on peut notamment citer les groupes aminométhyl, aminoéthyl, iso-propylamino, butylamino et pentylamino.

Selon une forme de réalisation préférée de l'invention, les composés de formule (I) sont choisis parmi ceux dans lesquels R₁, R₂ et R₃ représentent un radical alcoxy, et de préférence un radical méthoxy.

Selon un autre mode de réalisation préféré, R₄ et R₇ représentent un atome d'hydrogène ou de chlore, un groupe méthyle, éthyle ou isopropyle.

Selon un autre mode de réalisation avantageux, R₆ représente un atome d'halogène ou un groupe alcoxy ou aminoalkyle, lesdits groupes alcoxy ou aminoalkyle pouvant comprendre de 1 à 6 atomes de carbone. Plus préférentiellement, R₆ représente un atome de chlore ou de fluor, un radical méthoxy ou diméthylamino.

A titre de composés de formule (I), on peut en particulier citer :
- l'hydrochlorure de N-(N-(3-(4-benzoylbenzamido)-4-méthylphényl)carbamimidoyl)-3,4,5-triméthoxybenzamide de formule suivante : 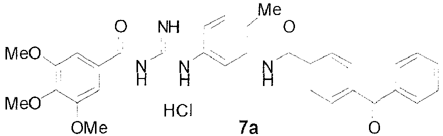 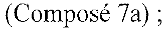
- l'hydrochlorure de *N-*(*N-*(3-(4-benzoylbenzamido)-4-méthylphényl)carbamimidoyl)-3,4,5-triméthoxybenzamide de formule suivante : 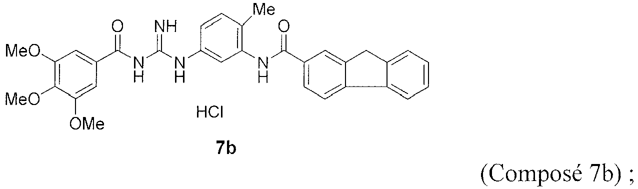
- l'hydrochlorure de 3,4,5-triméthoxy-*N*-(*N-*(4-méthyl-3-(4-(3-phénylpropyl)benzamido)phényl)carbamimidoyl) benzamide de formule suivante : 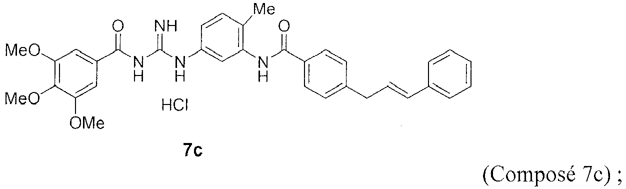
- l'hydrochlorure de 3,4,5-triméthoxy-*N-*(*N-*(4-méthyl-3-(4-phénétbylbenzamido)phényl)carbamimidoyl)benzamide de formule suivante : 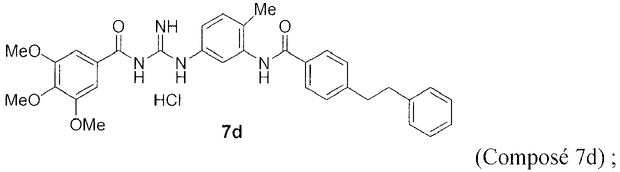
- l'hydrochlorure de (*E*)-*N-*(*N*-(3-(4-cinnamylbenzamido)-4-méthylphényl)carbamimidoyl)-3,4,5-triméthoxybenzamide de formule suivante : 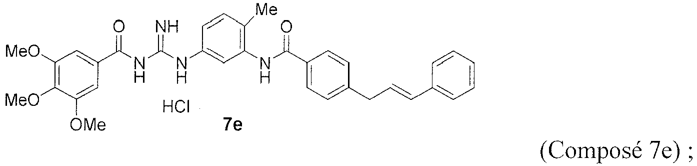
- l'hydrochlorure de *N-*(*N-*(3-(4-benzylbenzamido)-4-méthylphényl)carbamimidoyl)-3,4,5-triméthoxybenzamide de formule suivante : 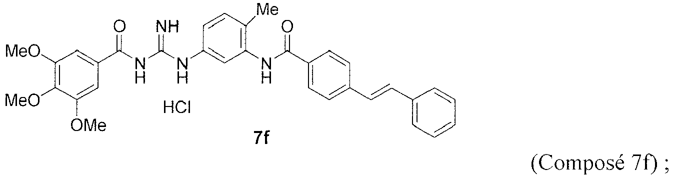
- l'hydrochlorure de *N-*(*N-*(3-(4-benzylbenzamido)-4-méthylphényl)carbamimidoyl)-3,4,5-triméthoxybenzamide de formule suivante : 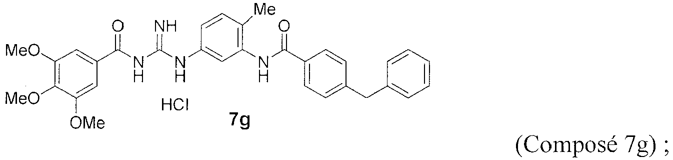
- l'hydrochlorure de *N-*(*N-*(*3-*(4-(benzyloxy)benzamido)-4-méthylphényl)carbamimidoyl)-3,4,5-triméthoxybenzamide de formule suivante :

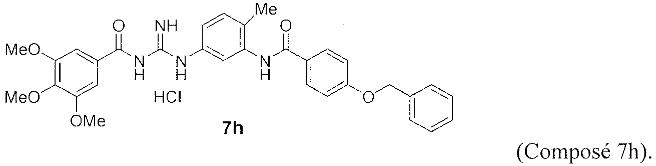

Les composés de formule (I) conformes à l'invention peuvent être facilement préparés, généralement en trois ou quatre étapes, selon des procédés de synthèse analogues aux procédés classiques connus de l'homme du métier.

Dans un premier temps un fragment central, le fragment (VI), est préparé selon le Schéma 1 ci-dessus.

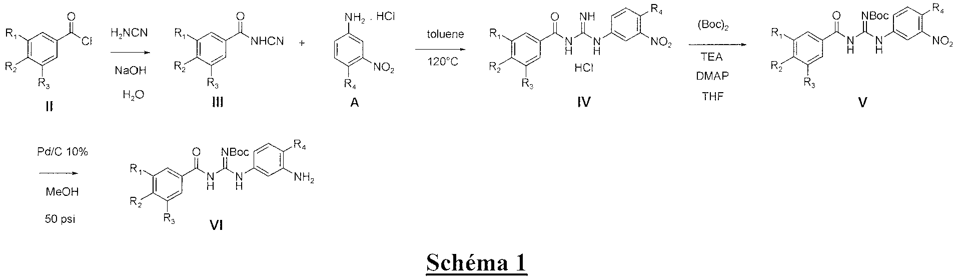

Il s'agit de condenser un chlorure de benzoyl (II) sur la cyanamide en milieu aqueux, de manière à obtenir une acyl-cyanamide (III), qui est à son tour condensé avec une aniline (A) pour obtenir la guanidine (IV).

La fonction basique de la guanidine est ensuite protégée par un résidu Boc en milieu basique, pour obtenir un composé nitro (V), puis réduite en amine par hydrogénation pour obtenir l'aniline intermédiaire (VI).

Le Schéma réactionnel 2 ci-après indique la préparation de composés possédant dans leur formule (I) des groupements R₁= R₂ =R₃ = MeO.

Les chlorures d'acides (1a-h) sont préparés selon les indications de la littérature (J. Med. Chem, 2001, 44, 3175 ; Chem. Eur. J. 2010, 16, 5848 ; Org. Lett. 2007, 9, 4571 ; Org. Biomol. Chem. 2008, 6, 3005 ; Adv. Cat. Synth. 2008, 350, 2065 ; J. Org. Chem. 2001, 66, 2874 ; J. Med. Chem. 2010, 53, 5770).

L'intermédiaire (VI) est ensuite condensé avec différents chlorures d'acide (1a-h) le plus souvent commerciaux pour fournir les composés attendus le plus souvent sous forme de sels (chlorhydrates) (Schéma 2).

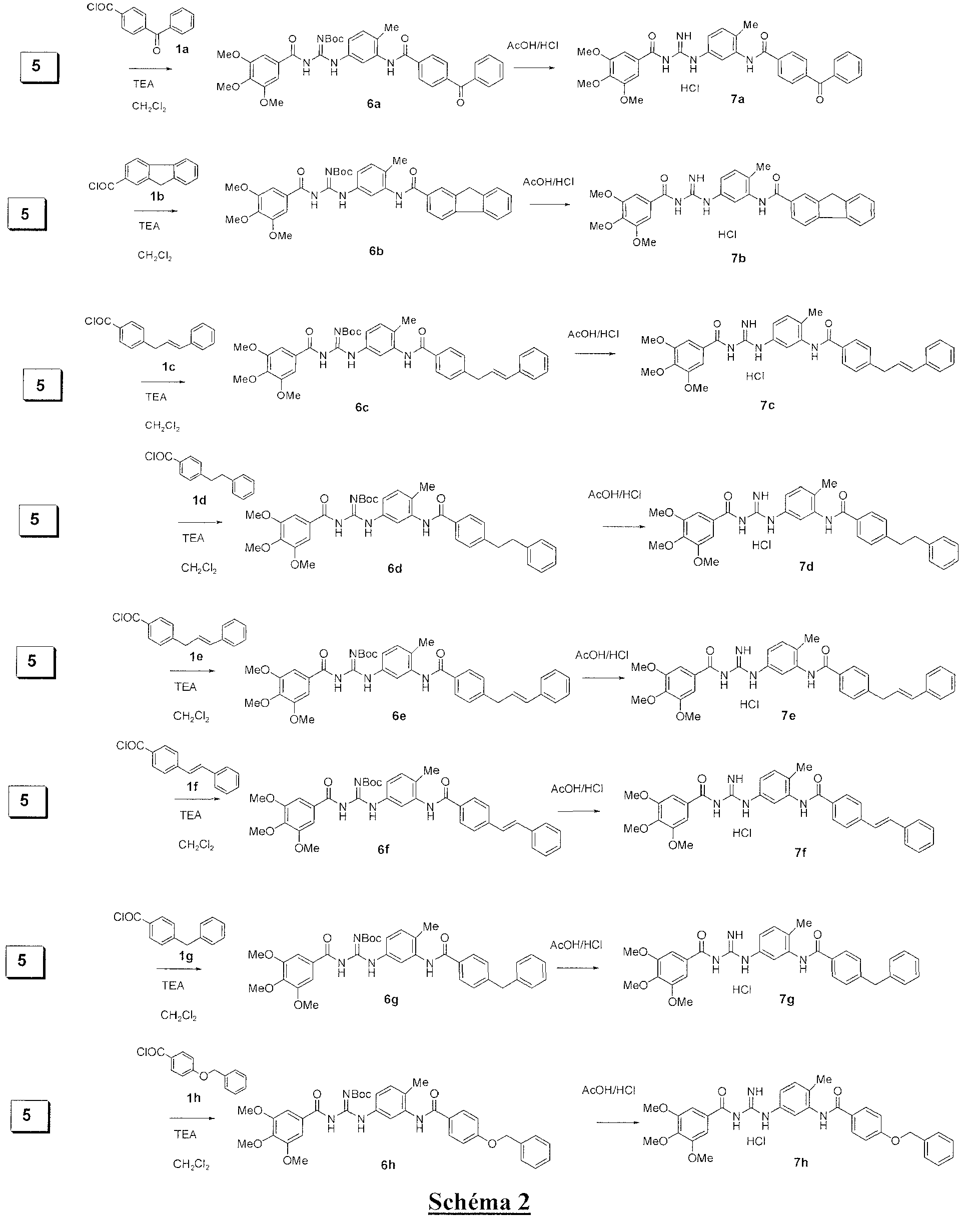

Les composés de formule (I) conformes à l'invention présentent la propriété de moduler négativement (effet inhibiteur) ou positivement (effet activateur) le voie de signalisation des protéines Hedgehog et peuvent donc être utilisés, à titre de principe actif, pour la préparation d'une composition pharmaceutique destinée aux traitements des pathologies associées à une hyperactivation ou à un déficit de la voie de signalisation des protéines Hedgehog.

Par conséquent, la présente invention a également pour objet des composés de formule (I), pour leur application en tant que médicament, et notamment des composés de formule (I) pour leur application en tant que médicament pour le traitement de tumeurs associées à une hyperactivation de la voie de signalisation des protéines Hedgehog.

Et plus particulièrement, la présente invention concerne également les composés de formule (I) :
- à titre de médicament destiné au traitement des tumeurs associées à une hyperactivation de la voie de signalisation des protéines Hedgehog ; de telles tumeurs sont notamment les tumeurs du tissu nerveux (médulloblastomes, tumeurs primitives neuroectodermiques, glioblastomes, méningiomes et oligodendrogliomes), des tumeurs cutanées (carcinomes basocellulaires, trichoépithéliomes), des tumeurs des tissus musculaires et osseux (rhabdomyosarcomes, ostéosarcomes) ou des tumeurs d'autres tissus (rein, vessie, prostate, poumon, estomac, pancréas),
- à titre de médicament pour le traitement de pathologies de type neuro-dégénératif comme la maladie de Parkinson, la Chorée de Huntington, la maladie d'Alzheimer, la sclérose en plaques ou la maladie du motoneurone,
- à titre de médicament pour le traitement de maladies liées au développement cérébral (holoprosencéphalie), au traitement d'accident vasculaire cérébral et aux accidents cardiovasculaires, ainsi qu'aux maladies des oligodentrocytes et des cellules de Schwann,
- pour une utilisation *in vitro* pour contrôler et moduler le renouvellement des cellules souches humaines ou animales,
- à titre de médicament pour le traitement d'autres pathologies dans lesquelles la modulation de la voie de signalisation Hedgehog pourrait être bénéfique, comme par exemple le diabète.

La posologie utile variera en fonction de l'affection à traiter, de la voie et du rythme d'administration, ainsi que de la nature et du poids de l'espèce à traiter (humaine ou animale) ; elle peut varier par exemple de 10 mg à 2 g par jour chez l'adulte par voie orale.

Un autre objet de la présente invention est une composition pharmaceutique, caractérisée par le fait qu'elle comprend, à titre de principe actif, au moins un composé de formule (I) tel que défini précédemment, et au moins un excipient pharmaceutiquement acceptable.

Au sein des compositions pharmaceutiques conformes à l'invention, le ou les composés de formule (I) sont de préférence utilisés en une quantité permettant d'administrer des doses unitaires comprises entre 10 mg et 2 g environ.

L'homme du métier choisira un ou plusieurs excipients pharmaceutiquement acceptables en fonction de la voie d'administration de la composition pharmaceutique. Bien entendu, l'homme de l'art veillera, à cette occasion, à ce que le ou les excipients utilisés soient compatibles avec les propriétés intrinsèques attachées à la composition conforme à la présente invention.

En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente invention, le médicament ou la composition pharmaceutique peut être administré par n'importe quelle voie appropriée, par exemple par la voie orale, anale, locale, systémique, intraveineuse, intramusculaire ou mucosale, ou bien en utilisant un patch, ou encore sous forme encapsulée dans, ou immobilisée sur, des liposomes, des microparticules, des microcapsules, et analogues.

On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc...

Les techniques de formulation et d'administration des médicaments et compositions pharmaceutiques sont bien connues dans la technique ici considérée, l'homme du métier pouvant notamment se référer à l'ouvrage Remington's Pharmaceutical Sciences, (21^{st} édition).

Les composés de formule (I) de l'invention s'avèrent être d'une grande utilité aussi bien comme outils pour la découverte de nouveaux modulateurs de ces voies de signalisation que pour déceler différentes formes du récepteur Smoothened ou de récepteurs apparentés et identifier des molécules capables de moduler ces différentes formes.

La présente invention se rapporte donc également à l'utilisation d'au moins un composé de formule (I) marqué comme outil de recherche, notamment pour l'identification de molécules capables d'interagir avec le récepteur Smoothened ou un récepteur apparenté, par exemple en se liant sur le site de liaison Smo1 ou sur le site de liaison Smo2.

En effet, les Inventeurs ont constaté de manière tout à fait inattendue que l'introduction d'un marqueur sur l'une des extrémités de fragments structurales des composés de formule (I) pouvait conduire à des radio-ligands présentant une affinité subnanomolaire caractérisée dans de nombreux tests biologiques.

Le marquage des composés de formule (I) peut être radioactif par incorporation d'isotopes radioactifs tels que ³H, ¹¹C, ¹⁴C, ³²P, ³⁵S, ¹²⁵I, ^{99m}Tc, ¹⁸F, ⁶⁴Cu, ⁷⁶Br, ¹²⁴I, ¹³N, ¹⁵O ou encore ¹²³I selon les méthodes classiques de l'état de la technique. Le marquage du composé de formule (I) peut aussi consister en la fixation d'un fluorophore sur ledit composé selon les méthodes connues de l'homme du métier ; la détection du marquage peut se faire classiquement à l'aide de radio-imageurs sélectionnés en fonction de l'atome radioactif à détecter, ou par mesure de la fluorescence.

Dans la littérature, plusieurs radio-ligands de la voie de signalisation ont été décrits, mais encore peu utilisés. Par exemple, les radio-ligands marqués au tritium ³H ou à l'iode ¹²⁵I comme le ³H SAG (Rominger et al., JPET, 2009, 995-1005), le ³H-Hh-Ag1.5 (Borzillo et Lippa., Curr. Top Med. Chem., 2005, 5(2), 147-57), le [³H]GDC-0449 (Dijkgraaf et al., 2011, Cancer Res. 435-444) ou la PA-cyclopamine [³H]-cyclopamine (Chen et al., Genes Dev., 2002, 16 : 2743-2748) :

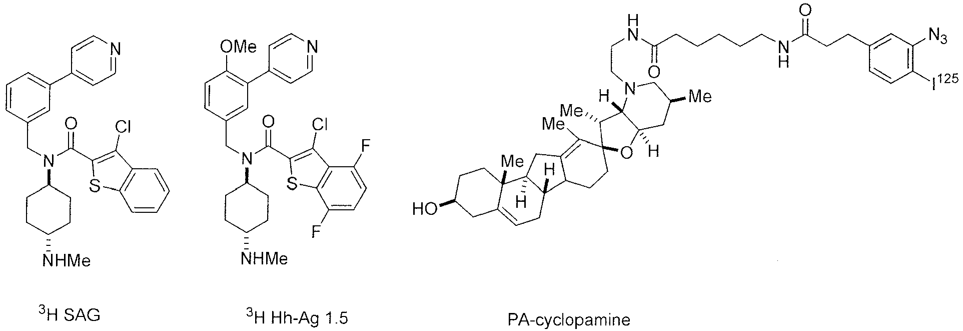

Une série d'analogues ont été étudiés, dans la perspective d'obtenir une substance marquée au tritium pour entreprendre des expériences de liaisons spécifiques et/ou d'autoradiographie pour analyser d'une manière approfondie le mécanisme d'action des modulateurs de récepteurs de la voie de signalisation des protéines Hedgehog, et plus particulièrement du récepteur Smoothened. En effet, les composés marquées précédemment décrits dans l'art antérieur ne sont pas faciles d'accès ; ils nécessitent un grand nombre d'étapes de synthèse. De plus, parmi les radio-ligands décrits ci-dessus, seule la PA-cyclopamine [³H]-cyclopamine est disponible commercialement. Elle présente en outre une faible affinité (Kd autour de 10 nM) (Rominger et al., JPET, 2009, 995-1005), contrairement aux composés de l'invention (Kd = 0.3 nM pour le composé (7d)).

Plus particulièrement, les composés de formule (I) sous forme marquée peuvent être utilisées :
- pour la détection de tumeurs associées à une hyperactivation de la voie de signalisation des protéines Hedgehog,
- pour le criblage et/ou l'identification de ligands de sites de liaison spécifiques des récepteurs de la voie de signalisation des protéines Hedgehog, tel que le récepteur Smoothened ou des récepteurs apparentés comme les récepteurs Frizzled (Frizzled 1 à 10), ou encore comme les récepteurs Patched (Patched 1 et Patched 2), Dispatched (Dispatched 1 et Dispatched 2) ou Hip, lesdits récepteurs de la voie de signalisation des protéines Hedgehog pouvant être exprimés de façon naturelle ou pouvant être transfectés (transfert de gène plasmidique) ou infectés (grâce à l'utilisation de virus) de façon stable ou transitoire dans des cellules primaires, des lignées cellulaires ou des tissus sains ou pathologiques,
- pour le criblage et/ou l'identification de nouvelles molécules antagonistes des récepteurs de la voie de signalisation des protéines Hedgehog (molécules anticancéreuses) ou d'agonistes des récepteurs de la voie de signalisation des protéines Hedgehog agissant sur les cellules souches,
- en autoradiographie pour analyser le fonctionnement de la voie de signalisation des protéines Hedgehog dans des cellules primaires, des lignées cellulaires ou des tissus sains ou pathologiques,
- pour étudier la régulation pharmacologique des récepteurs de la voie de signalisation des protéines Hedgehog dans des cellules primaires, des lignées cellulaires ou des tissus sains ou pathologiques.

Un autre objet de la présente invention concerne un procédé de radio-marquage d'un composé de formule (I) tel que défini ci-dessus comprenant une étape de tritiation d'un composé de formule (I) sous une atmosphère de tritium ³H, et ledit procédé comprenant une étape préalable d'halogénation lorsque le composé de formule (I) est substitué par un groupe R₅ exempt de double liaison éthylénique.

Ainsi, un objet supplémentaire de la présente invention concerne un composé de formule (I) susceptible d'être obtenu selon le procédé de radio-marquage décrit ci-dessus, dans lequel au moins un de ses atomes d'hydrogène a été remplacé par un atome de tritium ³H. Ces composés de formule (I) radio-marqués au tritium ³H peuvent être utilisés comme outils de recherche, et pour les différentes utilisations énumérées ci-dessus.

Diverses méthodes de criblage de modulateurs de la voie Hedgehog sont connues :
- méthodes utilisant des réponses cellulaires de lignées ou cultures primaires : différenciation cellulaire (C3H10T1/2), prolifération cellulaire (cellules primaires granulaires du cervelet) ;
- méthodes utilisant une compétition avec un composé fluorescent agissant sur les domaines transmembranaires du récepteur ciblé [(bodipycyclopamine (BC)] (US 2007/0218775) ;
- méthodes utilisant un gène rapporteur de la voie impliquée (Taipale et al., 2000, Nature, 406, 1005-1009) ;
- un essai détectant une activité Hh en mesurant les interactions Ptc-Hh en présence ou non de composé à tester (US 2005/0282231) ;
- un essai utilisant des cellules défectives pour Sufu et un rapporteur pour identifier des inhibiteurs de la voie Hh agissant en aval de Smo (WO 2006/080894).

La présente invention se rapporte aussi à une méthode de criblage et/ou d'identification de ligands des sites de liaison Smo (Smo1 ou Smo2) du récepteur Smoothened comprenant les étapes suivantes :
a) mise en contact du récepteur Smoothened et d'au moins un composé de formule (I) pour l'obtention d'un complexe [Smo-composé de formule (I)] ;
b) mise en contact du récepteur Smoothened, dudit composé de formule (I) et d'une molécule à tester ;
c) détection d'une interaction entre ledit récepteur Smoothened et lesdites molécules à tester par comparaison du récepteur Smoothened récupéré à l'étape b) avec le complexe [Smo-composé de formule (I)] ; et
d) sélection desdites molécules à tester pour lesquelles une interaction avec le récepteur Smoothened est mesurée.

L'étape a) peut être mise en oeuvre avec des cellules exprimant le récepteur Smoothened ou avec des extraits de membranes comprenant le récepteur Smoothened fonctionnel, il peut notamment s'agir d'extraits de membranes de levures portant le récepteur humain Smoothened fonctionnel susceptibles d'être obtenus avec le procédé décrit par de Rivoyre et al. (FEBS Letters 579, 2005, 1529-1533).

Lorsque l'étape a) est mise en oeuvre avec des cellules exprimant le récepteur Smoothened, l'expression dudit récepteur est soit constitutive (le récepteur est naturellement exprimé par la cellule), soit elle résulte de la transformation d'une cellule afin qu'elle exprime ou sur-exprime un récepteur Smoothened de la même espèce ou d'une espèce différente. Ce récepteur Smoothened peut aussi porter une mutation qui lui confère des propriétés biochimiques ou pharmacologiques différentes du récepteur sauvage.

Tout composé de formule (I) peut être utilisé pour la mise en oeuvre de la méthode selon l'invention ; il pourra toutefois être choisi par l'homme du métier notamment en fonction de son affinité pour le récepteur Smoothened.

L'étape a) peut être mise en oeuvre en phase liquide ou sur un support solide convenable ; l'homme du métier choisira et adaptera ces modalités de mise en oeuvre par exemple selon qu'il utilise le récepteur Smoothened sous la forme d'extraits de membranes ou sous la forme de protéine purifiée.

Lorsque l'étape a) est mise en oeuvre en phase liquide, la méthode de criblage en phase liquide peut être mise en oeuvre selon les étapes suivantes :
a) mise en contact du récepteur Smoothened et d'au moins un composé de formule (I) pour l'obtention d'un complexe [Smo-composé de formule (I)] ;
b) mise en contact du récepteur Smoothened, dudit composé de formule (I) et d'une molécule à tester ;
c) récupération dudit récepteur Smoothened éventuellement lié à une ou plusieurs molécules à tester et/ou audit composé de formule (I) ;
d) détection d'une interaction entre ledit récepteur Smoothened et lesdites molécules à tester par comparaison du récepteur Smoothened récupéré à l'étape c) avec le complexe [Smo-composé de formule (I)] ; et
e) sélection desdites molécules à tester pour lesquelles une interaction est mesurée.

L'étape a) peut être mise en oeuvre avec des extraits membranaires comprenant le récepteur Smoothened qui sont alors incubés avec le composé de formule (I) et les molécules à tester ; le mélange est ensuite centrifugé ; le culot obtenu après centrifugation est à nouveau suspendu dans un tampon, puis à nouveau centrifugé pour éliminer les interactions non spécifiques. La fixation de molécule à tester au récepteur Smoothened est alors analysée soit par mesure de fluorescence, soit par mesure de radioactivité suivant le marquage du composé de formule (I) :
- selon une variante de la méthode de criblage en phase liquide, ledit composé de formule (I) est préalablement marqué. L'étape d) de détection de l'interaction entre le récepteur Smoothened et une ou plusieurs des molécules à tester se fait par comparaison du marquage (par radioactivité ou fluorescence) des récepteurs Smoothened récupérés à l'étape c) avec celui du complexe [Smo-composé de formule (I)] ; les molécules sélectionnées sont celles pour lesquelles le marquage des récepteurs Smoothened récupérés à l'étape c) est plus faible que celui du complexe [Smo-composé de formule (I)] ;
- selon une autre variante de la méthode de criblage en phase liquide, la détection de l'interaction se fait par chromatographie en comparant la position du complexe [Smo-composé de formule (I)] avec celle du récepteur Smoothened récupéré à l'étape c), si la position est identique alors il n'y a pas d'interaction entre le récepteur Smoothened et la molécule à tester*. A contrario'* une différence de position indique une interaction, il convient ensuite de confirmer que l'interaction entre la molécule à tester et le récepteur Smoothened se produit bien sur un site de liaison Smo.

L'étape a) de la méthode de criblage selon l'invention peut alternativement être mise en oeuvre sur un support solide convenable.

Par support solide, on entend en particulier un biocapteur constitué d'une membrane qui comprend une espèce biologique, telle qu'une enzyme, un anticorps, un peptide, un micro-organisme, un tissu biologique, un lipide, un acide nucléique..., permettant la liaison avec une molécule à tester et un transducteur permettant de transformer le signal biologique, tel que la fixation d'un ligand sur un récepteur protéique, en un signal physique mesurable, par exemple électrochimique (ampérométrique, potentiométrique, conductimétrique), optique (lumineux), piézoélectrique ou calorimétrique. Dans le cas présent, l'espèce biologique fixée sur la membrane est le récepteur Smoothened ; dans deux expériences conduites en parallèle, le récepteur Smoothened sur support solide est mis en contact avec un composé de formule (I) et avec le mélange dudit composé de formule (I) et d'une molécule à tester, les signaux obtenus dans chacune de ces expériences sont comparés ; sont sélectionnées les molécules qui induisent une modification du signal.

A titre d'exemple, le support solide est un biocapteur de détection d'une liaison par résonance plasmonique dont l'utilisation permet de visualiser et de caractériser (constantes d'affinité, d'association et de dissociation) des interactions entre une protéine et son ligand par changement de masse à la surface du biocapteur. Ce changement de masse se mesure par des variations de l'angle de résonance plasmonique à la surface du biocapteur et ne nécessite pas de ligand marqué ou fluorescent.

Lorsque la méthode de criblage sur support solide est mise en oeuvre avec des extraits de membranes comprenant le récepteur Smoothened, ces extraits peuvent être fixés par injection sur un biocapteur lipophile. Lorsqu'un biocapteur lipophile est utilisé, les extraits membranaires se fixent aux groupements lipophiles liés par des liaisons covalentes au dextran, ce qui permet de suivre l'interaction entre récepteurs membranaires et ligands (M. R. Pourshafie et al., J. Microbiol. Meth., 2004, 58, 313-320; A. Wikstrom, Anal. Biochem., 2007, 362, 98-107).

Ainsi selon une variante de l'invention, le biocapteur de détection d'une liaison par résonance plasmonique est un biocapteur lipophile tel qu'un biocapteur hydrophobe « sensorchip L1 » de Biacore (GE Healthcare) sur lequel est fixée par injection une préparation de membranes contenant le récepteur Smoothened.

Lorsque la méthode de criblage sur support solide est mise en oeuvre avec le récepteur Smoothened purifié, ledit récepteur peut également être fixé sur un biocapteur permettant une mesure de résonance plasmonique de surface qui consiste à détecter une variation de l'indice de l'interface sur laquelle est fixé le récepteur lorsqu'un ligand s'y fixe.

Selon une autre variante, la méthode de criblage sur support solide permet également d'identifier des ligands du récepteur Smoothened qui ne se lient pas à un site de liaison Smo ; l'action activatrice ou inhibitrice de ces ligands peut être caractérisée par les méthodes qui suivent.

La présente invention se rapporte aussi à une méthode d'identification d'agonistes du récepteur Smoothened, qui, en plus des étapes décrites pour la méthode d'identification de ligand, comprend les étapes additionnelles de mise en contact du ligand identifié avec une cellule qui présente une réponse cellulaire suite à l'activation du récepteur Smoothened et de sélection des molécules agonistes capables d'induire ladite réponse cellulaire de ladite cellule.

Les cellules qui présentent une réponse cellulaire suite à l'activation du récepteur Smoothened sont choisies parmi les lignées ou cultures primaires : cellules mésenchymateuses (par exemple, C3H10T1/2) répondant à une activation du récepteur Smoothened par la différenciation cellulaire mesurable par l'activité de la phosphatase alcaline ; cellules primaires granulaires du cervelet répondant à une activation du récepteur Smoothened par une prolifération cellulaire ; cellules souches du cerveau adulte ou des progéniteurs neuraux ou encore des cellules progénitrices présentes dans les tissus au cours du développement ou chez l'adulte pour lesquelles la réponse cellulaire peut, par exemple, consister en l'induction de gènes tels que ceux codants pour les facteurs de transcription de la famille des Gli, ou encore pour Patched ou Hip, gènes activés par la voie Hedgehog.

La présente invention se rapporte également à une méthode d'identification d'une molécule antagoniste du récepteur Smoothened comprenant les étapes suivantes :
a) mise en culture de cellules qui présentent une réponse cellulaire suite à l'activation du récepteur Smoothened avec au moins un composé de formule (I) de façon à induire ladite réponse cellulaire ;
b) mise en contact des cellules obtenues à l'issue de l'étape a) avec une molécule à tester ;
c) sélection des molécules induisant une inhibition de ladite réponse cellulaire desdites cellules.

La molécule dont on veut tester l'activité antagoniste du récepteur Smoothened peut notamment être un ligand se liant sur un site de liaison Smo du récepteur Smoothened identifié par l'une quelconque des méthodes précédentes d'identification de ligand selon l'invention.

La présente invention se rapporte encore à un kit permettant la mise en oeuvre des méthodes selon l'invention comprenant au moins le récepteur Smoothened fonctionnel et au moins un composé de formule (I). Le récepteur Smoothened fonctionnel est soit présent sous forme d'extrait membranaire, soit dans des cellules qui présentent une réponse cellulaire suite à l'activation du récepteur Smoothened.

Les expériences de liaison entre le récepteur Smoothened et un composé de formule (I) peuvent également être utilisées pour caractériser et identifier :
- de nouveaux types cellulaires exprimant le récepteur Smoothened dans une conformation où le site de liaison Smo est actif ;
- des récepteurs impliqués dans la différenciation, tels que des récepteurs apparentés au récepteur Smoothened.

L'invention se rapporte alors à une méthode d'identification de cellules, telles que des cellules tumorales, exprimant le récepteur Smoothened comprenant les étapes de :
a) mise en contact de cellules à tester avec un composé de formule (I) marqué ;
b) nettoyage des cellules afin d'éliminer ledit composé de formule (I) marqué qui ne se serait lié à aucun récepteur des cellules à tester ;
c) détections des cellules marquées.

Les composés peuvent également servir à l'identification et à la caractérisation de nouveaux récepteurs ou de nouvelles formes de récepteurs impliqués dans la différenciation cellulaire, la prolifération, la mort cellulaire, la migration, la survie cellulaire ou encore permettant à la cellule d'acquérir une propriété ou un état qu'elle n'a pas encore atteint.

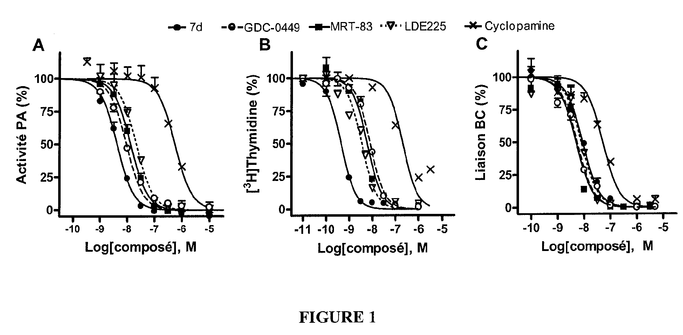

Outre les dispositions qui précèdent, l'invention comprend encore d'autres dispositions qui ressortiront de la description qui va suivre, qui se réfère à des exemples de synthèse, de caractérisation et d'évaluation de composés de formule (I) selon l'invention, et à leur marquage, ainsi qu'aux dessins annexés dans lesquels : la Figure 1 illustre l'activité du composé (7d) et des composés de référence dans différents tests cellulaires et sur la liaison de la bodipy-cyclopamine (BC) au récepteur Smo. Les Figures 1A et 1B montrent l'activité des composés sur la différenciation des cellules C3H10T1/2 (Figure 1A), et sur la prolifération des PCGs de cervelet de rat (Figure 1B). Les courbes d'inhibition ont été générées avec des concentrations croissantes des molécules étudiées en présence de SAG (0.1 µM, Figure 1A et 0.01 µM, Figure 1B). Les valeurs sont exprimées en pourcentage de la réponse maximale induite par le SAG. La Figure 1C illustre l'inhibition de la liaison de la bodipy-cyclopamine (BC) par les différents composés. Les cellules HEK-hSmo sont incubées avec la BC (5 nM) seule (contrôle) ou en présence d'une concentration croissante des molécules étudiées. La liaison de la BC est visualisée par microscopie à fluorescence et la courbe dose-réponse des composés est obtenue après quantification. Les valeurs sont exprimées en pourcentage de la liaison spécifique de la BC. Les données correspondent aux valeurs (moyennes ± SEM) issues d'une expérience représentative de 2-4 expériences indépendantes,

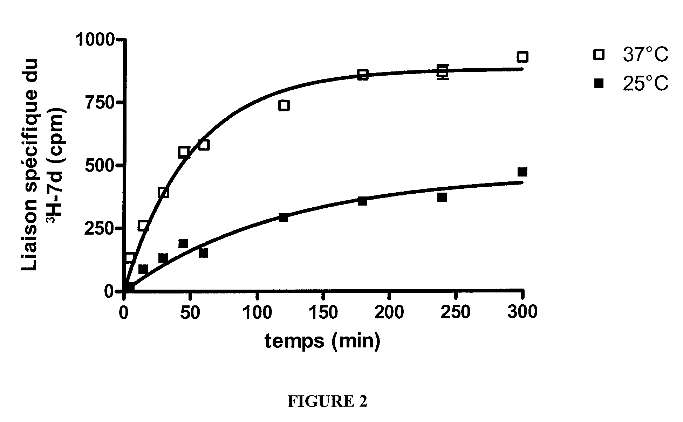

- la Figure 2 représente la cinétique d'association du composé ³H-(7d) à des membranes de cellules HEK-hSmo exprimant le récepteur Smoothened humain,

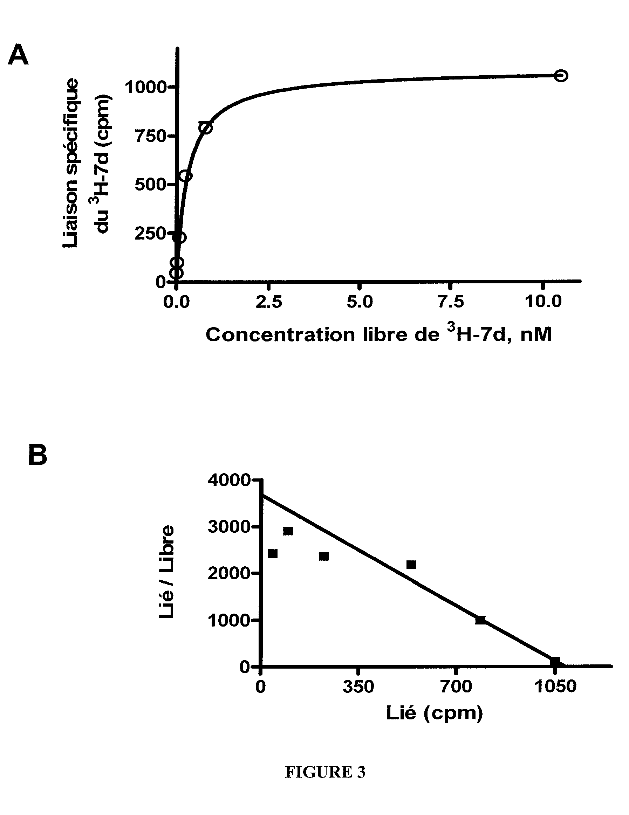

- la Figure 3 représente la courbe de saturation de la liaison du composé ³H-(7d) au récepteur Smo exprimé dans les cellules HEK-hSmo à l'équilibre. Figure 3A : Les membranes de cellules HEK-hSmo (2 µg de protéines) ont été incubées dans un volume final de 0.4 ml de tampon HEPES 0.2 % BSA avec des concentrations croissantes de composé 3H-(7d) pendant 3 heures à 37°C. La liaison non spécifique a été évaluée en présence de 1 µM de GDC-0449. L'analyse de la liaison spécifique donne des valeurs de K_{d} de 0.3 ± 0.1 nM et de Bₘₐₓ de 1086 ± 91 cpm. Les données sont les moyennes ± SEM de triplicats (expérience représentative, n = 3). Figure 3B : Analyse de Scatchard de la liaison spécifique du composé ³H-(7d),

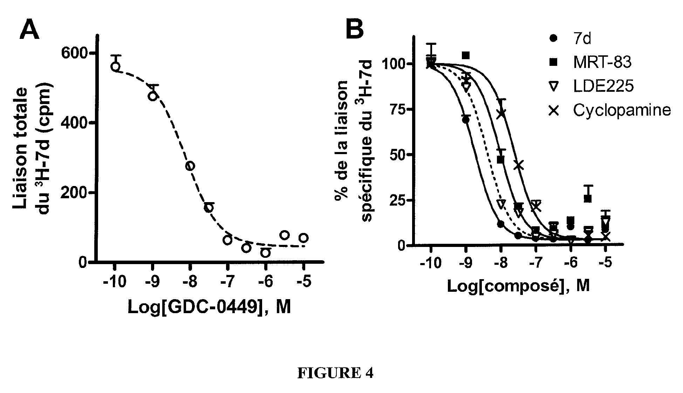

- la Figure 4 illustre l'inhibition de la liaison du composé ³H-(7d) au récepteur Smoothened humain exprimé dans les cellules HEK-hSmo. Les membranes de cellules HEK-hSmo (2 µg de protéines) ont été incubées pendant 3 heures à 37 °C dans un volume final de 0.4 ml de tampon HEPES (0.2 % BSA) avec 0.35 nM de composé ³H-(7d) seul ou en présence de concentrations croissantes de GDC-0449 (A), de composés (7d), MRT-83, LDE225 et de cyclopamine (B). Les données sont les moyennes ± SEM de triplicats (expérience représentative, n = 2-3) et représentent la liaison totale du composé ³H-(7d) (A) ou le pourcentage de la liaison spécifique du composé ³H-(7d) déterminée en présence de 1 µM de GDC-0449 (B),

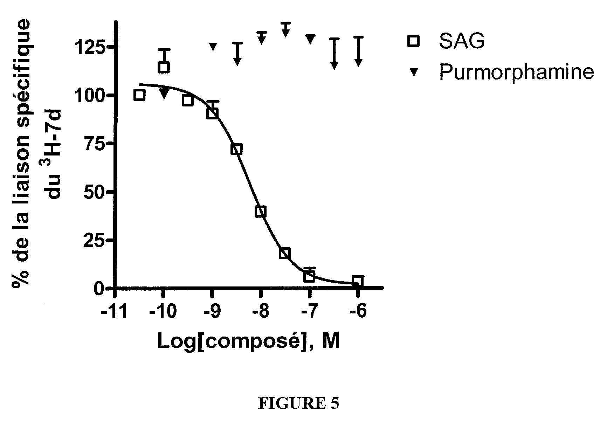

- la Figure 5 illustre l'inhibition de la liaison du composé ³H-(7d) au récepteur Smoothened humain exprimé dans les cellules HEK-hSmo par le SAG et la Purmorphamine. Les membranes de cellules HEK-hSmo (2 µg de protéines) ont été incubées dans un volume final de 0.4 ml de tampon HEPES (0.2 % BSA) avec 0.35 nM de composé ³H-(7d) seul ou en présence de concentrations croissantes de SAG ou de Purmorphamine pendant 3 heures à 37 °C. Les données sont les moyennes ± SEM de triplicats (expérience représentative, n = 2-3) et représentent le % de la liaison spécifique du composé ³H-(7d) déterminée en présence de 1 µM de GDC-0449.

### EXEMPLE 1 : PROTOCOLES DE SYNTHÈSE ET CARACTERISATION DE DIFFÉRENTS COMPOSÉS SELON L'INVENTION

Les réactions ont été conduites sous atmosphère de gaz inerte (azote) en utilisant des techniques de Schlenk (standard). Les solvants ont été séchés selon des méthodes standards et distillés sous azote avant utilisation. Tous les réactifs ont été obtenus dans le commerce et utilisés tels quels sans purification préalable.

Les spectrométries de masse (ESI+) ont été enregistrées sur un spectromètre LC/MSD vendu sous la référence Agilent^{®} 1100. Les spectres de résonance magnétique nucléaire (RMN) ont été enregistrés sur un appareil Bruker^{®} AC200 à 200 MHz (¹H) ou sur un appareil Bruker^{®} AC400 à 400 MHz (¹H).

### A/ Préparation d'un composé de formule IV

### Préparation de la 3,4,5-triméthoxy-N-(N-(4-méthyl-3 nitrophényl)carbamimidoyl)benzamide (3)

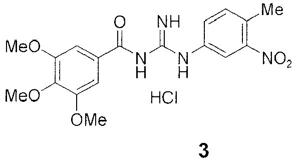

Une solution de cyanamide (2) (1.12 g, 4.77 mmol) et de 4-methyl-3-nitro aniline (A1) sous forme de chlorhydrate [1.08 g, 5.72 mmol, preparé par l'addition de 1 éq. de HCl (2M dans Et₂O) à l'aniline dans le MeOH (10 mL), suivi d'une concentration sous vide pour obtenir une poudre] dans du toluène (50 mL) est chauffé à reflux pendant 6 heures sous agitation. Il se forme un solide. De l'Et₂O (50 mL) est ajouté pour favoriser la precipitation. Le solide est filtré et séché sous vide ; puis le solide est dissout dans une solution aqueuse saturée de NaHCO₃ dans H₂O (30 mL). La solution aqueuse est extraite avec de l'Et₂O (2 x 50 mL). La phase organique est lavée avec de la saumure, séchée sur du Na₂SO₄ et concentrée pour obtenir la nitro-guanidine (3) (1.22 g, 66%).
¹H-NMR (CDCl₃, 300 MHz) d = 7.80 (brs, 1 H), 7.34-7.27 (m, 5 H), 3.90 (s, 9 H).
PM = 388 for C₁₈H₂₀N₄O₆ [ES/MS] m/z 389 [M + 1]⁺

### B/ Préparation d'un composé de formule V

### Préparation du tert-butyl (4-méthyl-3-nitrophenylamino) (3,4,5-triméthoxybenzamido) méthylène carbamate (4)

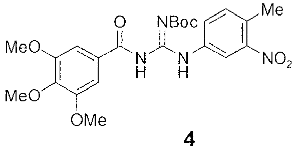

A une solution de guanidine sous forme de base (3) (3.16 g, 8.13 mmol) dans le THF (90 mL) est ajouté goutte à goutte une solution de Boc₂O (1.77g, 8.13 mmol) dans le THF (10 mL) à température ambiante et du DMAP (97 mg, 1 mmol) comme catalyseur. Après 1 heure d'agitation le composé de départ a été consommé. Le solvant est évacué sous vide, et le résidue purifié par chromatographie sur colonne en éluant avec un mélange AcOEt/Hept : 3/7. La Boc-guanidine est obtenue sous forme d'huile (4) (2.3 g, 58%).
¹H-NMR (CDCl₃, 300 MHz) d = 7.27 (s, 1 H), 7.09-7.04 (m, 3 H), 6.59 (m, 2 H), 3.82 (s, 3 H), 3.70 (s, 6 H), 2.17 (s, 4 H), 1.39 (s, 9 H).
PM = 488 for C₂₃H₂₈N₄O₈ [ES/MS] m/z 489 [M + 1]⁺

### C/ Préparation d'un composé de formule VI

### Préparation du tert-butyl (3-amino-4-méthylphénylamino) (3,4,5-triméthoxybenzamido) méthylène carbamate (5)

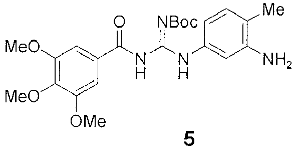

Dans un flacon, un mélange de Boc nitro-guanidine (4) (1.22 g, 2.5 mmol) dans du MeOH (80 mL) et du Pd/C 10% (100 mg) est agité sous H₂ à 50 psi pendant 10 heures. Le substrat de départ a disparu, le catalyseur est filtré et le solvant est évacué sous vide. Le résidu est purifié par chromatographie sur colonne en éluant avec un mélange d'Hept/AcOEt : 7/3 pour obtenir le compose titre comme huile jaune (5) (840 mg, 73%).
¹H-NMR (CDCl₃, 300 MHz) d = 7.27 (s, 1 H), 7.09-7.04 (m, 3 H), 6.59 (m, 2 H), 3.82 (s, 3 H), 3.70 (s, 6 H), 2.17 (s, 3 H), 1.39 (s, 9 H).
PM = 458 for C₂₃H₃₀N₄O₆ [ES/MS] m/z 459 [M + 1]⁺

### D/ Préparation de différents composé de formules (6) et (7)

### D- 1) Préparation du tert-butyl(3-(4-benzoylbenzamido)-4-méthylphénylamino) (3,4,5-triméthoxybenzamido) méthylènecarbamate (6a)

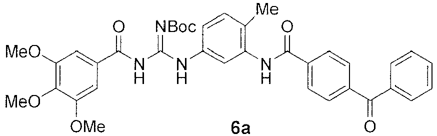

A une solution de Boc guanidine (5) (137 mg, 0.3 mmol) et de triéthylamine (0.1 mL, 0.66 mmol) dans du CH₂Cl₂ (10 mL) est ajouté goutte à goutte une solution de chlorure d'acide (1a) (73 mg, 0.3 mmol) dans du CH₂Cl₂ (3 mL) à 0°C. Le milieu réactionnel est agité durant une nuit. De l'eau (15 mL) et du CH₂Cl₂ (10 mL) sont ajoutés. La phase organique est lavé avec de la saumure, séchée et concentrée sous vide. Le résidu est purifié par chromatographie sur colonne sur gel de silice en éluant avec un mélange d'Hept/AcOEt : 3/2. Le compose titre est obtenu sous la forme d'un solide blanc (6a) (142 mg, 70%, pF 96°C).
¹H-NMR (CDCl₃, 300 MHz) d = 10.47 (brs, 1 H), 9.33 (brs, 1 H), 8.01-7.82 (m, 8 H), 7.55-7.53 (m, 3 H), 7.29-7.10 (m, 3 H), 3.82 (s, 3 H), 3.71 (s, 6 H), 2.41 (s, 3 H), 1.44 (s, 9 H).

### D- 2) Préparation de l'hydrochlorure de N-(N-(3-(4-benzoylbenzamido)-4-méthylphényl)carbamimidoyl)-3,4,5-triméthoxybenzamide (7a)

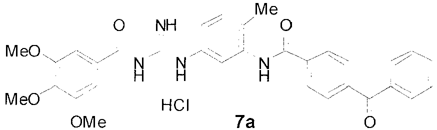

Une solution de Boc-guanidine (6a) (85 mg, 0.127 mmol) dans un mélange d'AcOH (1 mL) et de HCl concentré (0.5 mL) est mélangé durant une nuit. Le mélange de reaction est concentré sous vide, repris dans de l'Et₂O, et à nouveau concentré pour donner un solide blanc (7a) (72 m, 94%, pF 138°C).
¹H-NMR (MeOH, d4, 400 MHz) d = 8.16 (d, J = 8 Hz, 2 H), 7.93 (d, J = 8 Hz, 2 H), 7.85-7.34 (m, 9 H), 3.97 (m, 6 H), 3.89 (s, 3 H), 2.43 (s, 3 H).
PM = 566 for C₃₂H₃₀N₄O₆ [ES/MS] m/z 567 [M + 1]⁺

### D- 3) Préparation du tert-butyl (3-(9H-fluorène-2-carboxamido)-4-méthylphénylamino) (3,4,5-triméthoxybenzamido) methylènecarbamate (6b)

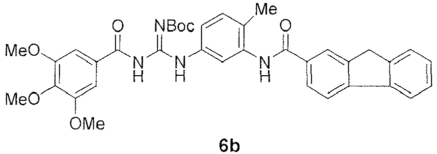

A une solution de Boc-guanidine (5) (137 mg, 0.3 mmol) et de triéthylamine (0.05 mL, 0.36 mmol) dans du CH₂Cl₂ (10 mL) du chlorure d'acide (1b) (82 mg, 0.3 mmol) en solution dans du CH₂Cl₂ (3 mL) est ajouté goutte à goutte. Le mélange est agité durant une nuit, puis de l'eau (5 mL) et du CH₂Cl₂ (10 mL) sont rajoutés. La phase organique est traitée par de la saumure, séchée et concentrée sous vide. Le résidu huileux est purifié par chromatographie sur colonne en éluant avec un mélange d'Hept/Et₂O : 3/2 pour obtenir le composé titre (6b) (109 mg, 56%).
¹H-NMR (CDCl₃, 300 MHz) d = 10.47 (bs, 1 H), 9.37 (m, 1H), 8.07-7.02 (m, 13 H), 3.99 (s, 2 H), 3.81 (s, 3 H), 3.71 (s, 6 H), 2.41 (s, 3 H), 1.43 (s, 9 H)

### D- 4) Préparation de l'hydrochlorure de N-(2-méthyl-5-(3-(3,4,5-triméthoxybenzoyl)guanidino)phényl)-9H-fluorène-2-carboxamide (7b)

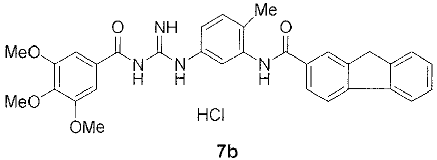

Le composé Boc (6b) (100 mg, 0.154 mmol) est mis en solution dans un mélange d'AcOH (1.1 mL) et de HCl concentré (0.55 mL). Le mélange est agité pendant 4 heures à température ambiante. Le solvant est évaporé sous vide et le résidu solide est repris par de l'Et₂O, puis filtré pour récupérer un solide blanc (7b) (72 mg, 80%, pF 241 °C)
¹H-NMR (DMSO, d6, 400MHz) d = 12.24 (brs, 1 H), 11.53 (brs, 1 H), 10.2 (s, 1 H), 9.42 (brs, 1 H), 8.95 (brs, 1 H), 8.23-7.40 (m, 12 H), 4.05 (s, 2 H), 3.91 (s, 6 H), 3.78 (s, 3 H), 2.35 (s, 3 H).
PM = 550 for C₃₂H₃₀N₄O₅ [ES/MS] m/z 551 [M + 1]⁺

### D- 5) Préparation du tert-butyl (4-méthyl-3-(4-(3-phénylpropyl)benzamido)phénylamino) (3,4,5-triméthoxybenzamido) méthylènecarbamate (6c)

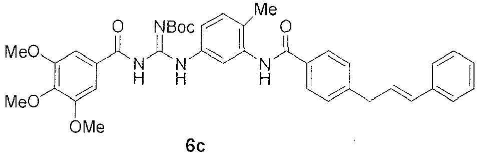

A une solution de Boc-guanidine (5) (137 mg, 0.3 mmol) et de triethylamine (0.1 mL, 0.66 mmol) dans du CH₂Cl₂ (10 ml) est ajoutée une solution du chlorure d'acide (1c) (73 mg, 0.3 mmol) dans du CH₂Cl₂ (3 mL) à température ambiante. Abandonner la réaction la nuit. Ajouter de l'eau (8 mL) et du CH₂Cl₂ (10 mL). Laver la phase organique avec une solution de NaCl saturée. Puis la phase organique est séchée et concentrée sous vide. Le résidu est purifié sur colonne sur gel de silice en éluant avec un mélange d'AcOEt/Hept : 2/3. Le composé titre est obtenu sous forme d'un solide blanc (6c) (132 mg, 65%).
¹H-NMR (CDCl₃, 300MHz) d = 10.47 (brs, 1 H), 9.37 (brs, 1 H), 8.02-7.01 (m, 18 H), 3.81 (s, 3 H), 3.69 (s, 6 H) 2.72-2.64 (m, 4 H), 2.05-1.92 (m, 2 H), 1.42 (s, 9 H)

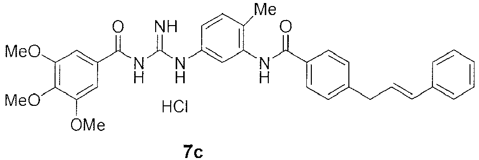

### D- 6) Préparation de l'hydrochlorure de 3,4,5-triméthoxy-N-(N-(4-méthyl-3-(4-(3-phénylpropyl)benzamido)phényl)carbamimidoyl) benzamide (7c)

Une solution du composé Boc (6c) (116 mg, 0.17 mmol) dans un mélange d'AcOH (1.5 mL) et de HCL concentré (0.75 mL) est agitée à température ambiante pendant 4 heures. Le solvant est ensuite évaporé et le résidu repris dans de l'Et₂O. Le composé titre est récupéré comme un solide blanc (80 mg, 82%, pF 132°C).
PM = 580 pour C₃₄H₃₆N₄O₅ [ES/MS] m/z 581 [M + 1]⁺

### D- 7) Préparation de tert-butyl (4-méthyl-3-(4-phénethylbenzamido)phénylamino) (3,4,5-triméthoxybenzamido) méthylène carbamate (6d)

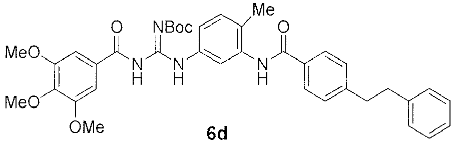

A une solution de Boc-guanidine utilisé comme base (5) (137 mg, 0.3 mmol) et de triéthylamine (0.06 mL, 0.45 mmol) dans du CH₂Cl₂ (10 mL) est ajouté une solution de chlorure d'acide (1d) (80 mg, 0.33 mmol) à température ambiante. La solution est agitée durant une nuit, et de CH₂Cl₂ est ajouté (10 mL), ainsi que de l'H₂O (10 mL). La phase organique est lavée avec de la saumure, séchée et concentrée sous vide. Le résidu est purifié par chromatographie sur colonne sur gel de silice en éluant avec de l'AcOEt/Hept : 2/3 pour obtenir le compose titre (6d) sous forme de solide (150 mg, 75%, pF 161°C).
¹H-NMR (CDCl₃, 300 MHz) d =10.47 (brs, 1 H), 9.37 (brs, 1 H), 8.02 -7.68 (m, 4 H), 7.29-7.01 (m, 11 H), 3.81 (s, 3 H), 3.68 (s, 6 H), 3.01-2.96 (m, 4 H), 2.37 (s, 3 H)1.42 (s, 9 H).
PM = 666 pour C₃₈H₄₂N₄O₇ [ES/MS] m/z 667 [M + 1]⁺

### D- 8) Préparation de l'hydrochlorure de 3,4,5-triméthoxy-N-N-(4-méthyl-3-(4-phénéthylbenzamido)phényl)carbamimidoyl)benzamide (7d)

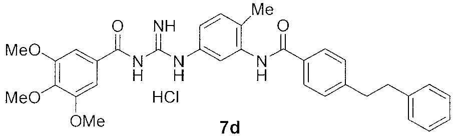

Une solution de l'adduit Boc (6d) (120 mg, 0.18 mmol) dans un mélange d'AcOH (1.3 mL) et de HCl concentré (0.66 mL) est agitée à température ambiante pendant 4 heures. Le solvant est évaporé sous vide, et le résidu repris avec de l'Et₂O (10 mL) et filtré pour obtenir une poudre blanche (7d) (101 mg, 93%, mp 186°C).
¹H-NMR (MeOH, d4, 300 MHz) d = 7.90 (d, 2 II J = 8 Hz), 7.52-7.16 (m, 12 H), 3.95 (s, 6 H), 3.87 (s, 3 H), 3.03-2.99 (m, 4 H), 2.38 (s, 3 H).
PM = 566 for C₃₃H₃₄N₄O₅ [ES/MS] m/z 567 [M + 1]⁺

### D- 9) Préparation du tert-butyl (3-(4-cinnamylbenzamido)-4-méthylphénylamino) (3,4,5-triméthoxybenzamido) méthylènecarbamate (6e)

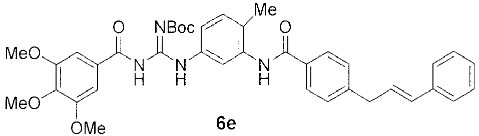

A une solution de Boc-guanidine (5) (137 mg, 0.3 mmol) et de triéthylamine (0.1 mL, 0.66 mmol) dans du CH₂Cl₂ (10 ml) est ajoutée une solution du chlorure d'acide (1e) (73 mg, 0.3 mmol) dans du CH₂Cl₂ (3 mL) à température ambiante. Abandonner la réaction la nuit. Ajouter de l'eau (8 mL) et du CH₂Cl₂ (10 mL). Laver la phase organique avec une solution de NaCl saturée. Puis la phase organique est séchée et concentrée sous vide. Le résidu est purifié sur colonne sur gel de silice en éluant avec un mélange d'AcOEt/Hept : 1/4. Le composé titre est obtenu sous forme d'un solide blanc (6e) (142 mg, 70%).
¹H-NMR (CDCl₃, 300MHz) d = 10.47 (brs, 1 H), 9.36 (brs, 1 H), 8.02-7.01 (m, 17 H), 6.51-6.32 (m, 2 H), 3.81 (s, 3 H), 3.69 (s, 6 H), 3.62 (m, 2 H), 2.36 (s, 3 H), 1.42 (s, 9 H)

### D- 10) Préparation de l'hydrochlorure de (E)-N-(N-(3-(4-cinnamylbenzamido)-4-méthylphényl)carbamimidoyl)-3,4,5-triméthoxybenzamide (7e)

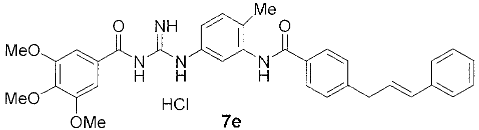

Une solution du composé Boc (6e) (122 mg, 0.15 mmol) dans un mélange d'AcOH (1.5 mL) et de HCL concentré (0.75 mL) est agitée à température ambiante pendant 4 heures. Le solvant est ensuite évaporé et le résidu repris dans de l'Et₂O. Le composé titre est récupéré sous forme de solide blanc (7e) (101 mg, 91%, pF 127°C).
¹H-NMR (MeOD, d4, 400MHz) d = 7.98-7.94 (m, 2 H), 7.52-7.28 (m, 12 H), 6.49-6.43 (m, 2 H), 3.95 (S, 6 H), 3.65 (s, 3 H), 3.66-3.64 (m, 2 H), 2.38 (s, 3 H).
PM = 578 for C₃₄H₃₄N₄O₅ [ES/MS] m/z 579 [M + 1]⁺

### D- 11) Préparation du tert-butyl (4-méthyl-3-(4-styrylbenzamido)phénylamino) (3,4,5-triméthoxybenzamido)méthylènecarbamate (6f)

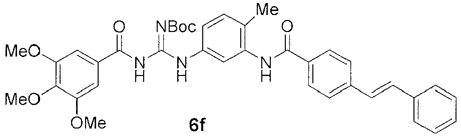

A une solution de Boc guanidine (5) (137 mg, 0.3 mmol) et de triéthylamine (0.1 mL, 0.66 mmol) dans du CH₂Cl₂ (10 mL) est ajoutée une solution du chlorure d'acide (1f) dans du CH₂Cl₂ (3 mL), puis le milieu réactionnel est laissé à réagir pendant une nuit. De l'eau (10 mL) et du CH₂Cl₂ (15 mL) sont ensuite ajoutés. La phase organique est décantée et lavée avec une solution de NaCl (saturée dans H₂O), séchée, évaporée et purifiée par chromatographie sur colonne de gel de silice en éluant avec un mélange d'AcOEt/Hept : 3/7, pour obtenir le composé titre sous forme d'un solide blanc (6f) (130 mg, 65%, Mp 166°C).
¹H-NMR (CDCl3, 300MHz) d = 10.47 (brs, 1 H), 9.35 (brs, 1 H), 8.02-7.09 (m, 17 H), 3.81 (s, 3 H), 3.71 (s, 6 H), 2.39 (s, 3 H), 1.43 (s, 9 H).

### D- 12) Préparation de l'hydrochlorure de N-(N-(3-(4-benzylbenzamido)-4-méthylphényl)carbamimidoyl)-3,4,5-triméthoxybenzamide (7f)

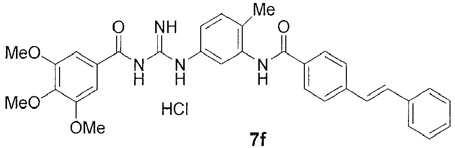

Une solution de composé Boc (6f) (120 mg, 0.18 mmol) dans un mélange d'AcOH (3 mL) et de HCl concentré (1.5 mL) est agitée à température ambiante pendant 2 heures. Le solvant est ensuite évaporé et le résidu repris avec de l'Et₂O pour obtenir un solide blanc (70 mg, 70%, pF 221 °C).
¹H-NMR (MeOD, d4, 300MHz) d = 8.02-7.29 (m, 16 H), 3.94 (s, 6 H), 3.87 (s, 3 H), 2.40 (s, 3 H).
PM = 564 for C₃₅H₃₂N₄O₅ [ES/MS] m/z 565 [M + 1]⁺

### D- 13) Préparation du tert-butyl (3-(4-benzylbenzamido)-4-méthylphénylamino) (3,4,5-trimethoxybenzamido) méthylènecarbamate (6g)

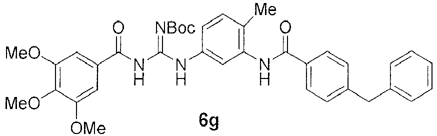

A une solution de Boc-guanidine (5) (137 mg, 0.3 mmol) et de triéthylamine (0.1 mL, 0.66 mmol) dans du CH₂Cl₂ (10 ml) est ajoutée une solution du chlorure d'acide (1g) (73 mg, 0.3 mmol) dans du CH₂Cl₂ (3 mL) à température ambiante. Le mélange est laissé à réagir durant une nuit. De l'eau (8 mL) et du CH₂Cl₂ (10 mL) sont ensuite ajoutés. La phase organique est lavée avec une solution de NaCl saturée, puis séchée et concentrée sous vide. Le résidu est purifié sur colonne sur gel de silice en éluant avec un mélange d'AcOEt/Hept : 1/8. Le composé titre est obtenu sous forme d'un solide blanc (6g) (144 mg, 71%, pF 105°C).
¹H-NMR (CDCl₃, 300MHz) d = 10.47 (brs, 1 H), 9.35 (brs, 1 H), 8.01-7.07 (m, 16 H), 4.06 (s, 2 H), 3.80 (s, 3 H), 3.68 (s, 6 H), 2.31 (s, 3 H).

### D- 14) Préparation de l'hydrochlorure de N-(N-(3-(4-benzylbenzamido)-4-méthylphényl)carbamimidoyl)-3,4,5-triméthoxybenzamide (7g)

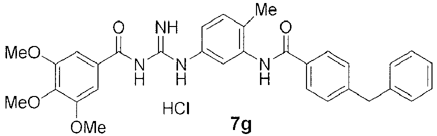

Une solution du composé Boc (7e) (103 mg, 0.15 mmol) dans un mélange d'AcOH (1.5 mL) et de HCL concentré (0.75 mL) est agitée à température ambiante pendant 4 heures. Le solvant est ensuite évaporé et le résidu repris dans de l'Et₂O. Le composé titre est récupéré sous forme de solide blanc (7g) (85 mg, 90%, pF 112°C).
¹H-NMR (MeOD, d4, 400MHz) d = 7.95 (d, J = 8 Hz, 1 H), 7.53-7.23 (m, 12 H), 4.09 (s, 2 H), 3.36 (s, 6 H), 3.86 (s, 3 H), 2.39 (s, 3 H).
PM = 552 for C₃₂H₃₂N₄O₅ [ES/MS] m/z 553 [M + 1]⁺

### D- 15) Préparation du tert-butyl (3-(4-(benzyloxy)benzamido)-4-méthylphénylamino) (3,4,5-triméthoxybenzamido)méthylènecarbamate (6h)

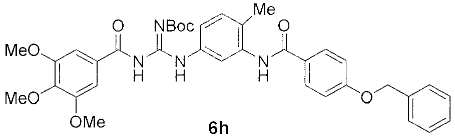

A une solution de Boc-guanidine (5) (137 mg, 0.3 mmol) et de triéthylamine (0.1 mL, 0.66 mmol) dans du CH₂Cl₂ (10 ml) est ajoutée une solution du chlorure d'acide (1h) (73 mg, 0.3 mmol) dans du CH₂Cl₂ (3 mL) à température ambiante. Le mélange est laissé à réagir durant une nuit. De l'eau (8 mL) et du CH₂Cl₂ (10 mL) sont ensuite ajoutés. La phase organique est lavée avec une solution de NaCl saturée, puis séchée et concentrée sous vide. Le résidu est purifié sur colonne sur gel de silice en éluant avec un mélange d'AcOEt/Hept : 1/4. Le composé titre est obtenu sous forme d'un solide blanc (6h) (103 mg, 68%, pF 94°C).

### D- 16) Préparation de l'hydrochlorure de N-(N-(3-(4-benzyloxy)benzamido)-4-méthylphényl)carbamimidoyl)-3,4,5-triméthoxybenzamide (7h)

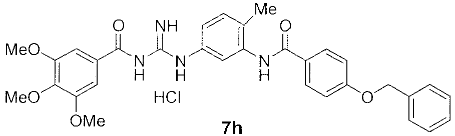

Une solution du composé Boc (6h) (116 mg, 0.18 mmol) dans un mélange d'AcOH (1.5 mL) et de HCL concentré (0.75 mL) est agitée à température ambiante pendant 4 heures. Le solvant est ensuite évaporé et le résidu repris dans de l'Et₂O. Le composé titre est récupéré sous forme de solide blanc (7h) (80 mg, 82%, pF 192°C).
¹H-NMR (MeOH, d6, 300MHz) d = 8.02-7.99 (m,2 H), 7.54-7.16 (m, 11 H), 5.23 (s, 2 H), 3.97 (s, 6 H), 3.88 (s, 3 H), 2.39 (s, 3 H)
PM = 568 for C₃₂H₃₂N₄O₆ [ES/MS] m/z 569 [M + 1]⁺

### E/ Radio-marquage de composés de formule (I)

### E- 1) Hydrogénation du composé (7f) en composé (7d)

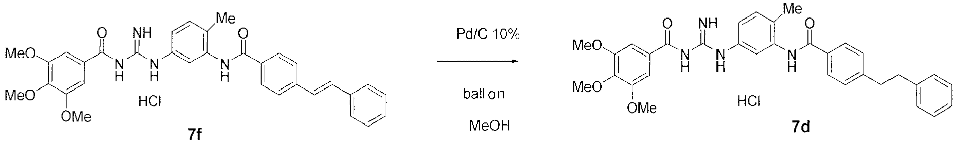

Une solution du composé (7f) sous forme de sel de chlorhydrate (5 mg, 0.08 mmol) dans du MeOH et du Pd/C (10%) est agitée sous hydrogène (ballon) pendant 4 heures. Le mélange est ensuite filtré et concentré sous vide pour obtenir le composé (7d) sous forme de sel de chlorhydrate.
PM = 566 for C₃₃H₃₄N₄O₅ [ES/MS] m/z 567 [M + 1]⁺ et 565 [M-1]⁺

### E- 2) Tritiation du composé (7f) en ³H-(7d)

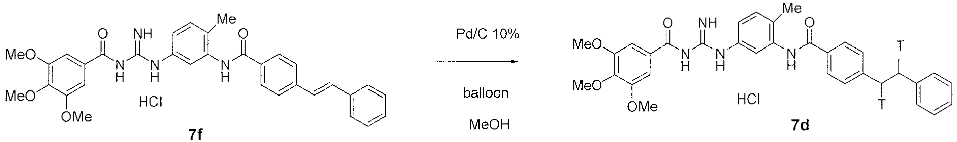

Dans un ballon de 5 mL le composé (7f) (2.5 mg, 0.04 mmol) est dissout dans du MeOH (1 mL) et la solution est refroidi (azote liquide). Le catalyseur (Pd/C 10%) est dispersé à la surface. Une fois le vide fait dans le ballon, du tritium gaz est introduit jusqu'à atteindre une pression de 30 psi. Le mélange réactionnel est agité pendant 3 heures. Le catalyseur est filtré et l'excès de tritium est évacué sous vide avec du MeOH. Le produit (7d) est obtenu directement sans purification.

### Caractéristique du composé [³H]-(7d) :

- Pureté : > 98% (HPLC)
- Activité Spécifique : 38.1 Ci/mmol (1.41 TBq/mmol)
- Concentration : 1.0 mCi/ml (37 MBq/ml)

### Données chromatographiques :

- Colonne HPLC : Macherey + Nagel Nucleodur Gravit)' C8, (5 flm), 4.6 x 150 mm
- Phase mobile : A : eau 0.05% TFA ; B : MeCN 0.05% TFA
- Gradient : 0 min 30 % B; 10 min 95% B; 14 min 95% B; 14 min 95% B; 14.5 min 30 %
- Débit : 1.0 ml/min
- Echantillon : 1,30 mCi/ml en Methanol (48 .1 MBq/ml)
- Injection : 5.0 µl (6.5 µCi, 240 KBq)
- Détection UV : 254 nm
- Température : 30°C
- Radio-détecteur : Berthold LB 513
- Cocktail: Zinsser Quicksz int Flow 302
- Débit : 2.0 mL/min
- Temps de rétention : 7.17 min (UV) ; 7.30 min (radio-détecteur) (le décalage entre les deux temps de rétention est du à l'installation en série des 2 détecteurs).

### EXEMPLE 2 : MISE EN ÉVIDENCE DE L'EFFET MODULATEUR DES COMPOSÉS DE FORMULE (I) SUR LA VOIE DE SIGNALISATION DES PROTÉINES HEDGEHOG ET DE LEUR FIXATION SUR LE RECEPTEUR SMOOTHENED.

L'effet des composés de formule (I) conformes à l'invention sur l'inhibition de la voie de signalisation des protéines Hedgehog a été déterminé *in vitro* par analyse de la différenciation de la lignée de cellules fibroblastiques pluripotentes C3H10T1/2 après activation de cette voie dans ces cellules par un activateur synthétique : le SAG. L'activité du composé X a également été évaluée sur la croissance d'une culture primaire de cellules granulaires du cervelet. La capacité de ce dernier composé à se lier au récepteur Smoothened de souris à également été déterminée par compétition avec la body-cyclopamine, un composé fluorescent dérivé de la cyclopamine et se fixant sur les domaines transmenbranaires du récepteur, comme décrit par Chen et al., Genes Dev., 2002, 16, 2743.

### 1- Matériels et méthodes

### Inhibition de la voie Hedgehog par les composés de formule (I) sur les cellules C3H10T1/2 :

Les composés de formule (I) à tester ont été dissous dans le diméthylsulfoxyde jusqu'à une concentration de 10 mM, puis stockés à une température de - 20°C jusqu'à utilisation.

La lignée de cellules fibroblastiques pluripotentes C3H10T1/2 (ATCC) a été cultivée dans les conditions recommandées par l'ATCC. L'activation de ces cellules a été réalisée en utilisant 0.1 µM de SAG selon les méthodes décrites par Chen et al., Proc. Natl. Acad. Sci. USA, 2002, 99, 14071 et Frank-Kamenetsky et al., J. Biol., 2002, 1, 10.

L'activation par le SAG provoque la différenciation de la lignée cellulaire et leur permet d'exprimer la phosphatase alcaline (PA). On a ainsi pu mesurer l'activité de la voie de signalisation des protéines Hedgehog via la mesure de l'activité phosphatase alcaline (PA).

Les cellules C3H10T1/2 ont été ensemencées sur des plaques de 96 puits à une densité de 5.10³ cellules par puits, 24 heures avant l'addition des composés à tester à une concentration variant de 1 nM à 30 µM et en présence de 0.1 µM de SAG, en utilisant comme milieu de culture du DMEM (Dulbecco's Modified Eagles's Medium) avec 10% de sérum de veau foetal. Les essais ont été réalisés en quadruplate. Les plaques ont ensuite été incubées pendant 5 à 6 jours à une température de 37°C sous atmosphère à 5% de CO₂. Les cellules ont ensuite été lavées dans du tampon phosphate froid («Phosphate Buffer Sérum» : PBS), puis lysées par sonication à 4°C dans 50 µL d'une solution contenant 0.9% de NaCl et 0.2% de Triton X-100.

A titre comparatif, l'activité d'autres inhibiteurs connus de la voie de signalisation des protéines Hedgehog ont été testés dans les mêmes conditions que celles utilisées pour tester les différents composés de formule (I) conformes à l'invention :
- la cyclopamine, telle que décrite par Incardona et al., Development, 1998, 125, 3553, et répondant à la formule suivante : 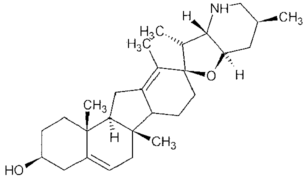
- le MRT-83 décrit ci-dessus, et
- le LDE225, tel que décrit par Pan et Dorsch ; ACS Med. Chem. Lett., 2010, 1 : 130-134, et répondant à la formule suivante :

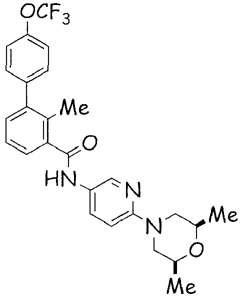

La mesure de l'activité phosphatase alcaline (PA) dans les lysats ainsi obtenus a ensuite été réalisée selon la méthode décrite par Pepinsky et al. (J. Biol. Chem., 1998, 273, 14037). Après addition de 100 µL de tampon réactionnel (200 mM Tris-HCl ; pH 10,5 ; 0,4 M de 2-amino-2-méthylpropanol et 8 mM de MgCl₂) et de 50 µL de substrat (4 mM de p-nitrophényl phosphate disodium), les lysats ont été incubés à 37°C pendant 30-60 min, puis la densité optique a été lue à une longueur d'onde de 415 nm.

### Activité des composés de formule (I) sur la prolifération des précurseurs des cellules granulaires de cervelet:

Les précurseurs des cellules granulaires (PCG) sont isolés à partir de cervelets de rats (IFFA- CREDO, France), 8 jours après la naissance (P8). Les cervelets sont prélevés, coupés en petits morceaux, placés dans le tampon de Krebs-Ringer (120 mM NaCl, 5 mM KCl, 1 mM KH₂PO₄, 25 mM NaHCO₃, 15 mM de glucose, 0.04 mM rouge de phénol) et incubés pendant 15 minutes à température ambiante dans un tampon de dissociation composé de Krebs-Ringer additionné de 250 mg/ml de trypsine (Sigma, France). La dissociation enzymatique est arrêtée par addition d'un volume égal de tampon Krebs-Ringer contenant 250 mg/ml d'un inhibiteur de trypsine et de 80 mg/ml de DNase (Sigma, France). Le tissu est centrifugé à 100 g pendant 10 secondes et le culot obtenu est remis en suspension et trituré à l'aide de pipettes pasteur de diamètre décroissant pour obtenir une suspension de cellules isolées. Cette suspension est centrifugée à 200 g pendant 5 minutes et le culot obtenu est remis en suspension dans du milieu Neurobasal supplémenté par 1 mM de pyruvate, 2 mM L-glutamine, pénicilline/streptomycine et 1% de supplément N2, 60 mg/ml N-acétyl cystéine et 100 mg/ml d'albumine de sérum bovin (BSA, Sigma, France).

Les PCG de cervelet obtenus sont transférés en plaques 96 puits préalablement traitées à la poly-D-lysine à une densité de 2.10⁵ cellules/puits. Le SAG en présence ou non des autres composés testés est ajouté immédiatement. 12 heures avant la fin de la culture, la thymidine tritiée (³H-Thymidine) est ajoutée. Les cellules sont aspirées et récupérées sur un filtre en fibre de verre (GF/C) en utilisant un collecteur de cellules automatisé (Brandel, USA). La quantité de radioactivité incorporée par les cellules a été quantifiée en présence de scintillant dans un compteur à scintillation liquide (Wallac, USA).

### Compétition des composés de formule (I) avec la bodipy-cyclopamine (BC):

Des cellules de la lignée de HEK293 transfectées de façon stable avec le récepteur Smo humain sont utilisées. L'expérience a été menée selon le protocole décrit dans Roudaut et al., Mol. Pharmacol. 79 : 453-460, 2011. L'inhibition de la liaison de la bodipy-cyclopamine (BC) par les composés de formule (I) est mesurée par la décroissance de fluorescence photographiée et quantifiée à l'aide du logiciel Simple PCI 6.2 (Hamamatsu Corporation), puis rapportée à la surface des noyaux présents sur la photographie.

### Test de radio-liaison du composé tritié ³H-(7d):

Le test de liaison se fait sur des membranes enrichies en récepteur Smoothened. Nous avons utilisé des cellules de la lignée de HEK-hSmo pour préparer ces membranes. Les cellules sont lavées et récupérées dans du PBS glacé. Après une centrifugation à 100 g pendant 7 minutes à 4°C, le culot est repris dans 10 fois son volume d'un tampon A glacé (50 mM HEPES, pH 7.4, 1 mM EDTA) additionné de 10 µl d'un cocktail d'inhibiteurs de protéases (PIC, Sigma) par ml de tampon, puis il est homogénéisé avec un broyeur Polytron. Après une centrifugation à 500 g pendant 30 minutes à 4°C qui permet d'éliminer les noyaux des cellules, le surnageant est à nouveau centrifugé à 48 000 g pendant 45 min à 4°C. Le culot est repris dans 2 ml de tampon A additionné de PIC puis la suspension est homogénéisée avec un broyeur conique en verre et passée à travers une aiguille de 23 G. Enfin, elle est aliquotée en tubes eppendorf et stockée à -80°C. La concentration de protéine totale de la préparation est déterminée par la méthode de Lowry utilisant la BSA pour réaliser la gamme étalon. Cette concentration est de 10.9 mg/ml.

Les membranes sont à nouveau suspendues dans un tampon (50 mM HEPES et 3 mM MgCl₂) contenant 0.2 % de BSA. Les expériences de liaison du ³H-(7d) sont réalisées dans des tubes en polypropylène. Les membranes (2 µg de protéines) sont incubées avec le ³H-(7d) en présence ou non des composés froids à tester pendant 3 heures à 37°C dans un volume final de 400 µl. L'incubation est stoppée en plongeant les tubes dans de l'eau glacée suivie d'une filtration rapide (Brandel) à travers une membrane de filtration fibres de verre (GF/C) traitée au préalable avec 0.3% de polyéthylenimine qui permet de diminuer la liaison non spécifique du ³H-(7d) à la membrane de filtration. La radioactivité retenue sur le filtre est mesurée en présence de 3 ml de scintillant dans un compteur à scintillation liquide. La liaison spécifique est définie comme la liaison pouvant être inhibée par 1 µM de GDC-0449 tel que décrit par Romer et al., Cancer Cell, 2004, 6, 229 et répondant à la formule suivante :

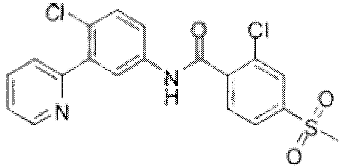

### 2- Résultats sur l'activité biologiques des composés de formule (I)

### Inhibition de la voie Hedgehog par les composés de formule (I) sur les cellules C3H10T1/2:

Des résultats obtenus avec des composés de formule (I) et les composés de référence cyclopamine, LDE225 et MRT-83 sont reportés dans le Tableau 1 ci-après. Pour chacun des composés la concentration qui permet d'inhiber 50% de l'activité phosphatase alcaline (PA) (CI₅₀) après induction par le SAG à 0.1 µM a été évaluée. Les courbes d'inhibition obtenues avec le composé (7d) et les composés de référence sont montrées à la Figure 1A.

**Tableau 1 : Activité des composés sur la différenciation des cellules C3H10T1/2.**

| Composés | CI₅₀ (nM) |
|---|---|
| Cyclopamine* | 620 ± 30 |
| GDC-0449* | 10 ± 1 |
| LDE225* | 22 ± 3 |
| MRT-83* | 11 ± 3 |
| 7a | 32 ± 11 |
| 7b | 28 ± 3 |
| 7c | 39 ± 9 |
| 7d | 6 ± 1 |
| 7e | 57 ± 2 |
| 7f | 15 ± 4 |
| 7g | 220 ± 25 |
| 7h | 72 ± 15 |

| | |
|---|---|
| * : Composés de référence ne faisant pas partie de l'invention | |

L'activité du composé (7d) a également été comparée au composé MRT-10 et à différents composés thiourés (composés 20 à 27 ci-dessous). Les résultats sont résumés dans le Tableau 2.

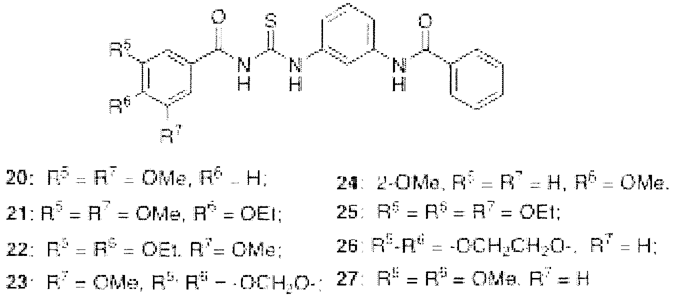

**Tableau 2 : Activité comparées des composés (7d), MRT-10 et de composés thiourés sur la différenciation des cellules C3H10T1/2.**

| C3H10T1/2, (SAG 0.1 µM) | |
|---|---|
| Composé | CI₅₀, µM |
| **7d** | 0.006 ± 0.001 |
| **MRT-10** | 0.9 ± 0.2 |
| **20** | 1.9 ± 0.2 |
| **21** | 0.6 ± 0.1 |
| **22** | 0.8 ± 0.2 |
| **23** | 1.1 ± 0.6 |
| **24** | 4.1 ± 0.3 |
| **25** | 0.8 ± 0.1 |
| **26** | inactif |
| **27** | > 5 |

| | |
|---|---|
| Moyenne ± SEM : n ≥ 3 | |

### Détermination de l'affinité des molécules sélectionnées sur la prolifération des précurseurs des cellules granulaires de cervelet de rat induite par le SAG :

Les PCGs du cervelet prolifèrent en réponse à l'activation de la voie de signalisation Shh, une réponse qui peut être inhibée par les antagonistes de Smo (Rohatgi et al., 2009, Proc. Natl. Acad. Sci. USA 106 : 3196-201). Nous avons donc analysé la capacité du composé (7d) à inhiber la prolifération des PCGs de cervelet de rat en culture primaire par une mesure d'incorporation de thymidine tritiée, un marqueur de l'ADN néosynthétisé au cours du processus de prolifération. Des concentrations croissantes de SAG provoquent une augmentation dose-dépendante de l'incorporation de la thymidine tritiée au dessus du niveau basal. Le composé (7d) présente des propriétés d'antagoniste complet avec une CI₅₀ de 0.45 nM sur la prolifération des PCGs induite par le SAG (0.01 µM) (Figure 1 B). Le LDE225, le composé MRT-83 et la cyclopamine bloquent également cette prolifération mais avec une affinité plus faible, leur CI₅₀ est de 3, 6 et 103 nM respectivement (Tableau 3).

### Détermination de l'affinité des molécules sélectionnées sur la liaison de la bodipy-cyclopamine (BC) au récepteur Smoothened :

Pour étudier les propriétés de liaison du composé (7d) à Smo, nous avons analysé si cette molécule pouvait entrer en compétition avec la liaison de la bodipycyclopamine (BC) (b) qui interagit avec Smo au niveau de ces domaines transmembranaires. Les cellules sont incubées pendant 2 heures avec la BC (5 nM) en présence ou en absence de différentes concentrations de composé (7d), MRT-83, LDE225 et cyclopamine. A la fin de l'incubation, les cellules sont fixées et colorées avec du DAPI, une molécule fluorescente de haute affinité pour l'ADN qui permet de visualiser les noyaux en bleu en microscopie à fluorescence. Les quatre molécules testées bloquent la liaison de la BC aux cellules HEK-hSmo, qui exprime de manière stable le récepteur Smo humain, de façon dose-dépendante. On observe que l'affinité du composé (7d) et du MRT-83 restent très proche et élevée (Figure 1 C). Toutes ces données démontrent que le composé (7d) est un antagoniste puissant des récepteurs Smo humain et de souris (Tableau 1).

**Tableau 3 : Activités comparées des composés (7d), MRT-83, LDE225, GDC-0449 et de la cyclopamine.**

| | CI₅₀ ± SEM (nM) | | |
|---|---|---|---|
| | Cellules C3H10T1/2 (2) | PCGs de cervelet de rat (3) | Liaison BC (4) |
| Composé (7d) | 6 ± 1 | 0.4 ± 0.1 | 8 ± 1 |
| MRT-83 | 11 ± 3 | 6 ± 1 | 5 ± 3 |
| LDE225 | 22 ± 3 | 3 ± 1 | 8 ± 2 |
| GDC-0449 | 10 ± 3 | 8 ± 1 | 5 ± 2 |
| Cyclopamine | 620 ± 30 | 103 ± 18 | 53 ± 11 |

Les CI₅₀ ont été déterminées par mesure de l'activité du rapporteur Gliluciférase induite par ShhN (4 nM) dans les cellules Shh-Light2 (1), de l'activité de la phosphatase alcaline (PA) associée à la différenciation des cellules C3H10T1/2 induite par le SAG (0.1 µM) (2), de l'activité proliférative des PCGs de cervelet de rat induite par le SAG (0.01 µM) suivie par l'incorporation de la ³H-Thymidine (3), de la liaison de la bodipy-cyclopamine (BC) au récepteur Smo humain exprimé dans les cellules HEK-hSmo (4) (d'après les courbes de la Figure 1).

L'ensemble des expériences réalisées met en lumière la capacité des composés de formule (I) à moduler la voie Shh *in vitro*. Leur activité pourrait s'expliquer par une liaison à la protéine Smoothened sur un site concurrent de la bodipy-cyclopamine (BC).

### 3- Résultats sur les caractéristiques de liaison du radio-ligand ³H-(7d)

### Cinétique d'association du radio-ligand ³H-(7d) au récepteur Smoothened humain :

Le composé (7d) est un antagoniste très puissant de la voie de signalisation Hh, ce qui nous a encouragés à synthétiser la forme tritiée de cette molécule : le composé ³H-(7d). Nous avons commencé la caractérisation des propriétés de ce radio-ligand en étudiant la cinétique d'association de la liaison du composé ³H-(7d) à des homogénats de membranes de cellules HEK293 exprimant de manière stable le récepteur Smo humain (HEK-hSmo). L'association du composé ³H-(7d) a été examinée en présence d'une concentration fixe du ligand radioactif (0.35 nM) et d'une quantité fixe de récepteur (2 µg de protéines) à 25°C et 37°C pendant 5 heures. Dans cette expérience, il s'agit de déterminer le temps nécessaire pour que la liaison spécifique du composé ³H-(7d) atteigne l'état d'équilibre. A 25°C, après 5 heures d'incubation, l'équilibre n'est pas atteint. A 37°C, l'équilibre est atteint après 3 heures d'incubation et se maintient après 5 heures d'incubation (Figure 2). Au-delà de 5 minutes d'incubation, la liaison non spécifique du composé ³H-(7d) (définie en présence de 0.1 µM de GDC0449) est faible (0.4 % de la liaison totale) et reste stable tout au long de l'expérience.

Les membranes de cellules HEK-hSmo (2 µg de protéines) ont été incubées dans un volume final de 0.4 ml de tampon HEPES (0.2 % BSA) avec 0.35 nM de ³H-(7d) pendant 5 h à 25°C (bleu) ou 37°C (rose). Les liaisons non spécifiques ont été évaluées en présence de 1 µM de GDC-0449, un antagoniste de référence de Smo. L'analyse par GraphPad Prism de la liaison spécifique donne un temps de demi-association de 33 min à 37°C et de 71 min à 25°C. Les données sont les moyennes ± SEM de triplicats (expérience représentative, n=2).

### Expérience de saturation de la liaison du composé ³H-(7d) au récepteur Smoothened humain :

Les propriétés du composé ³H-(7d) ont ensuite été caractérisées en étudiant la saturation de sa liaison au récepteur Smo exprimé dans les membranes des cellules de la lignée HEK-hSmo. Il s'agit de mesurer la liaison spécifique à l'équilibre, lors de l'incubation de concentrations croissantes du ligand radioactif, avec une quantité fixe de récepteur (2 µg de protéines). La saturation de la liaison spécifique du composé ³H-(7d) au récepteur Smo exprimé dans les membranes des cellules HEK-hSmo, définie en utilisant 1 µM de GDC-0449, est illustrée à la Figure 3A. L'analyse de la courbe indique un seul site de liaison de haute affinité : K_{d} = 0.3 ± 0.1 nM et Bₘₐₓ = 1086 ± 91 cpm visualisés par la représentation de Scatchard (Figure 3B).

Afin de s'assurer que la liaison du composé ³H-(7d) était bien spécifique au récepteur Smo, nous avons également réalisé cette expérience de saturation avec des membranes de cellules HEK293 non transfectées par l'ADNc codant pour le récepteur Smo humain. Le GDC-0449 (1 µM) n'a pas d'effet sur la liaison du composé ³H-(7d) à ces membranes. La liaison du composé ³H-(7d) aux membranes des cellules HEK-hSmo est donc bien spécifique du récepteur Smo.

### Analyse pharmacologique de la liaison du composé ³H-(7d) au récepteur Smoothened humain :

La pharmacologie de la liaison du composé ³H-(7d) au récepteur Smo exprimé dans les membranes des cellules de la lignée HEK-hSmo a été analysée en présence d'antagonistes et d'agonistes de Smo. Dans cette expérience, le ligand froid entre en compétition avec le ligand radioactif pour sa liaison au récepteur, et l'affinité (Kᵢ) de chaque ligand froid pour le récepteur peut être analysée à partir des CI₅₀ déduites des courbes d'inhibition. Le composé (7d) froid est le plus puissant pour inhiber la liaison du composé ³H-(7d) à Smo (CI₅₀ = 1.5 nM). Les composés LDE225, GDC-0449 et MRT-83 présentent des valeurs de CI₅₀ respectives de 4 nM, 12 nM et 11 nM. La concentration de 1 µM de GDC-0449 inhibe complètement la liaison spécifique du composé ³H-(7d). La liaison non spécifique a été définie pour chaque expérience en présence de 1 µM de GDC-0449. La cyclopamine et le composé Cur61414 sont également capables d'inhiber la liaison du composé ³H-(7d) mais avec une affinité très inférieure, notamment pour le Cur61414 avec une valeur de CI₅₀ de l'ordre du micromolaire (Figure 4). Pour les agonistes, le SAG présente une affinité de l'ordre du nanomolaire (CI₅₀ = 5 nM) alors que la Purmorphamine n'est pas active (CI₅₀ > 1000) (Figure 5). Les valeurs des Kᵢ calculées pour les composés sont présentées dans le Tableau 3.

**Tableau 4 : Affinités de molécules de référence vis-à-vis de la liaison du composé ³H-(7d) au récepteur Smoothened humain**

| | Liaison du composé ³H-(7d) | |
|---|---|---|
| | CI₅₀ ± SEM (nM) | Kᵢ ± SEM (nM) |
| Composé (7d) | 1.5 ± 0.2 | 0.7 ± 0.1 |
| MRT-83 | 10.5 ± 2.5 | 4.8 ± 1.2 |
| LDE225 | 4.2 ± 0.8 | 1.9 ± 0.3 |
| Cyclopamine | 30 ± 4 | 14 ± 2 |
| GDC-0449 | 12 ± 5 | 6 ± 2 |
| SAG | 5.2 ± 0.8 | 2.4 ± 0.3 |
| Purmorphamine | inactive | inactive |

Les valeurs de CI₅₀ et Kᵢ ont été déterminées par mesure de la liaison du composé ³H-(7d) au récepteur Smo humain exprimé dans les cellules de la lignée HEK-hSmo. Les données sont les moyennes ± SEM de 2-3 expériences représentatives.

## Revendications

1. Composé **caractérisé en ce qu'**il répond à la formule (I) suivante : 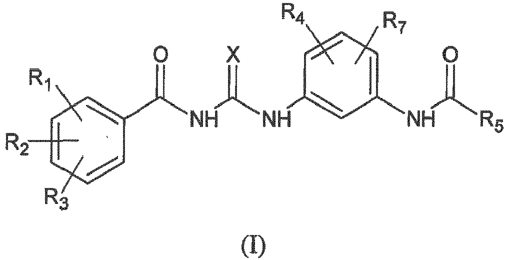 dans laquelle :
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy, alkylthio ou nitrile, et de préférence R₁, R₂ et R₃ représentent un radical alcoxy, et encore plus préférentiellement un radical méthoxy,
- X représente O, S ou NH, et de préférence X est NH,
- R₄ et R₇, identiques ou différents et indépendamment l'un de l'autre, représentent un atome d'hydrogène ou d'halogène ou un groupe alkyle, et de préférence R₄ et R₇ représentent un atome d'hydrogène ou de chlore, un groupe méthyle, éthyle ou isopropyle,
- R₅ représente un des groupes choisis parmi : 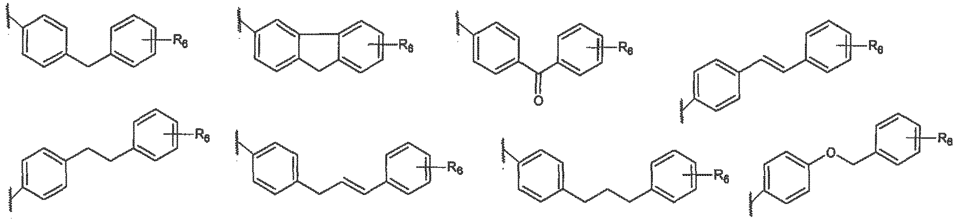 substitué par au moins un radical R₆ représentant un atome d'halogène ou un groupe alkyle, alcoxy, aminoalkyle, thioalkyle ou hydroxyle, et de préférence R₆ représente un atome d'halogène ou un groupe alcoxy ou aminoalkyle.

2. Composé de formule (I) selon la revendication 1, **caractérisé en ce qu'**il est choisi parmi :
- l'hydrochlorure de *N*-(*N*-(3-(4-benzoylbenzamido)-4-méthylphényl)carbamimidoyl)-3,4,5-triméthoxybenzamide de formule suivante : 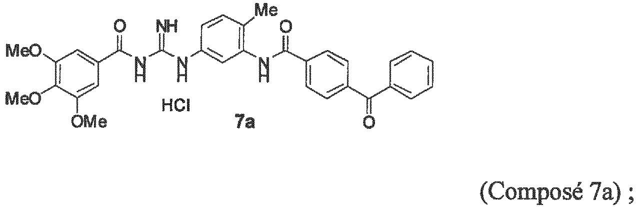
- l'hydrochlorure de *N-*(*N-*(3-(4-benzoylbenzamido)-4-méthylphényl)carbamimidoyl)-3,4,5-triméthoxybenzamide de formule suivante : 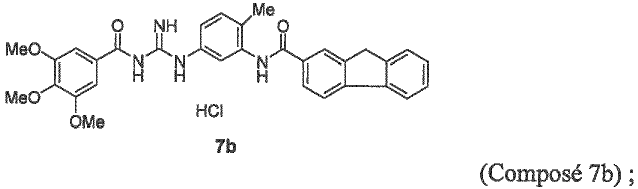
- l'hydrochlorure de 3,4,5-triméthoxy-*N*-(*N*-(4-méthyl-3-(4-(3-phénylpropyl)benzamido)phényl)carbamimidoyl) benzamide de formule suivante : 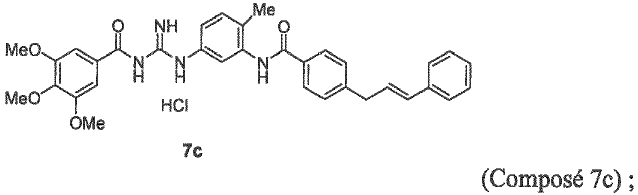
- l'hydrochlorure de 3,4,5-triméthoxy-*N*-(*N*-(4-méthyl-3-(4-phénéthylbenzamido)phényl)carbamimidoyl)benzamide de formule suivante : 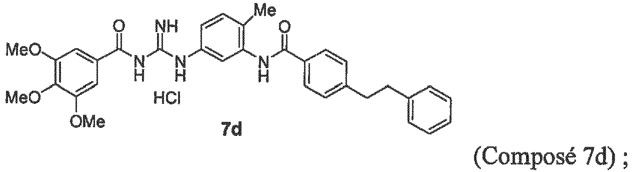
- l'hydrochlorure de (*E*)-*N*-(*N*-(3-(4-cinnamylbenzamido)-4-méthylphényl)carbamimidoyl)-3,4,5-triméthoxybenzamide de formule suivante : 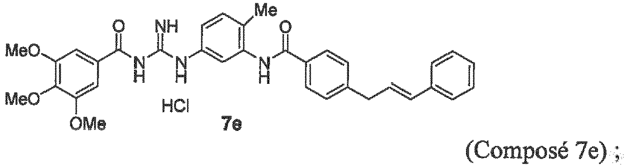
- l'hydrochlorure de *N*-(*N*-(3-(4-benzylbenzamido)-4-méthylphényl)carbamimidoyl)-3,4,5-triméthoxybenzamide de formule suivante : 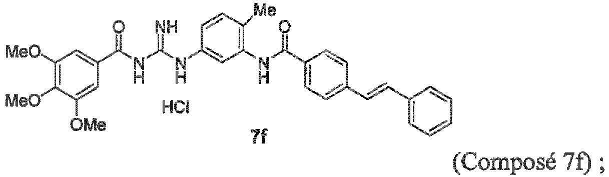
- l'hydrochlorure de *N*-(*N*-(3-(4-benzylbenzamido)-4-méthylphényl)carbamimidoyl)-3,4,5-triméthoxybenzamide de formule suivante : 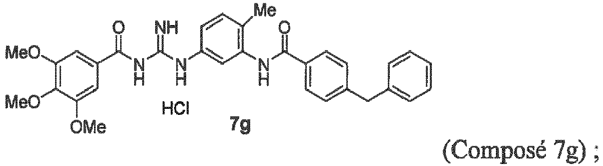
- l'hydrochlorure de *N*-(*N*-(3-(4-(benzyloxy)benzamido)-4-méthylphényl)carbamimidoyl)-3,4,5-triméthoxybenzamide de formule suivante :

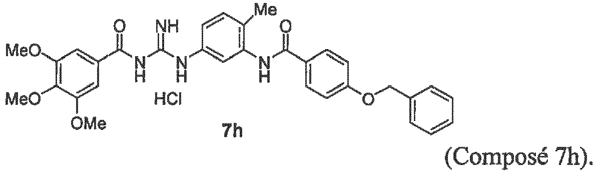

3. Composé de formule (I) selon la revendication 1 ou la revendication 2, pour son application en tant que médicament.

4. Composé de formule (I) selon la revendication 3, pour son application en tant que médicament pour le traitement de tumeurs associées à une hyperactivation de la voie de signalisation des protéines Hedgehog, et de préférence pour le traitement des tumeurs du tissu nerveux (médulloblastomes, tumeurs primitives neuroectodermiques, glioblastomes, méningiomes et oligodendrogliomes), des tumeurs cutanées (carcinomes basocellulaires, trichoépithéliomes), des tumeurs des tissus musculaires et osseux (rhabdomyosarcomes, ostéosarcomes) ou des tumeurs d'autres tissus (rein, vessie, prostate, poumon, estomac, pancréas).

5. Composé de formule (I) selon la revendication 3, pour son application en tant que médicament pour le traitement de pathologies de type neuro-dégénératif, et de préférence pour le traitement de la maladie de Parkinson, la Chorée de Huntington, la maladie d'Alzheimer, la sclérose en plaques ou la maladie du motoneurone.

6. Composé de formule (I) selon la revendication 3, pour son application en tant que médicament pour le traitement de maladies liées au développement cérébral (holoprosencéphalie), au traitement d'accident vasculaire cérébral et aux accidents cardiovasculaires, ainsi qu'aux maladies des oligodentrocytes et des cellules de Schwann.

7. Composé de formule (I) selon la revendication 3, pour son application *in vitro* pour moduler le renouvellement des cellules souches humaines ou animales.

8. Composé de formule (I) selon la revendication 3, pour son application en tant que médicament pour le traitement du diabète.

9. Composition pharmaceutique **caractérisée en ce qu'**elle comprend, à titre de principe actif, au moins un composé de formule (I) selon la revendication 1 ou la revendication 2 et au moins un excipient pharmaceutiquement acceptable.

10. Utilisation d'un composé de formule (I) marqué comme outil de recherche, notamment pour l'identification de molécules capables d'interagir avec le récepteur Smoothened ou un récepteur apparenté.

11. Procédé de radio-marquage d'un composé de formule (I) selon la revendication 1 ou la revendication 2, **caractérisé en ce qu'**il comprend une étape de tritiation d'un composé de formule (I) sous une atmosphère de tritium ³H, et ledit procédé comprenant une étape préalable d'halogénation lorsque le composé de formule (I) est substitué par un groupe R₅ exempt de double liaison éthylénique.

12. Composé de formule (I) selon la revendication 1 ou la revendication 2, **caractérisé en ce qu'**au moins un de ses atomes d'hydrogène a été remplacé par un atome de tritium ³H, et susceptible d'être obtenu selon le procédé de la revendication 11.

13. Méthode de criblage et/ou d'identification de ligands des sites de liaison Smo du récepteur Smoothened, **caractérisée en ce qu'**elle comprend les étapes suivantes :
a) mise en contact du récepteur Smoothened et d'au moins un composé de formule (I) pour l'obtention d'un complexe [Smo-composé de formule (I)] ;
b) mise en contact du récepteur Smoothened, dudit composé de formule (I) et d'une molécule à tester ;
c) détection d'une interaction entre ledit récepteur Smoothened et lesdites molécules à tester par comparaison du récepteur Smoothened récupéré à l'étape b) avec le complexe [Smo-composé de formule (I)] ; et
d) sélection desdites molécules à tester pour lesquelles une interaction avec le récepteur Smoothened est mesurée.

14. Méthode de criblage et/ou d'identification selon la revendication 13, **caractérisée en ce que** l'étape a) est mise en oeuvre en phase liquide, et **en ce que** ladite méthode comprend les étapes suivantes :
a) mise en contact du récepteur Smoothened et d'au moins un composé de formule (I) pour l'obtention d'un complexe [Smo-composé de formule (I)] ;
b) mise en contact du récepteur Smoothened, dudit composé de formule (I) et d'une molécule à tester ;
c) récupération dudit récepteur Smoothened éventuellement lié à une ou plusieurs molécules à tester et/ou audit composé de formule (I) ;
d) détection d'une interaction entre ledit récepteur Smoothened et lesdites molécules à tester par comparaison du récepteur Smoothened récupéré à l'étape c) avec le complexe [Smo-composé de formule (I)] ; et
e) sélection desdites molécules à tester pour lesquelles une interaction est mesurée.

15. Méthode d'identification d'agonistes du récepteur Smoothened, **caractérisée en ce qu'**elle comprend les étapes de l'une des méthodes des revendications 13 ou 14, et les étapes additionnelles suivantes :
- mise en contact du ligand identifié avec une cellule qui présente une réponse cellulaire suite à l'activation du récepteur Smoothened, et
- sélection des molécules agonistes capables d'induire ladite réponse cellulaire de ladite cellule.

16. Méthode d'identification d'une molécule antagoniste du récepteur Smoothened, **caractérisée en ce qu'**elle comprend les étapes suivantes :
a) mise en culture de cellules qui présentent une réponse cellulaire suite à l'activation du récepteur Smoothened avec au moins un composé de formule (I) de façon à induire ladite réponse cellulaire ;
b) mise en contact des cellules obtenues à l'issue de l'étape a) avec une molécule à tester ;
c) sélection des molécules induisant une inhibition de ladite réponse cellulaire desdites cellules.

17. Méthode d'identification de cellules, telles que des cellules tumorales, exprimant le récepteur Smoothened, **caractérisé en ce qu'**il comprend les étapes suivantes :
a) mise en contact de cellules à tester avec un composé de formule (I) marqué ;
b) nettoyage des cellules afin d'éliminer ledit composé de formule (I) marqué qui ne se serait lié à aucun récepteur des cellules à tester ;
c) détections des cellules marquées.

18. Kit **caractérisé en ce qu'**il comprend au moins un récepteur Smoothened fonctionnel et au moins un composé de formule (I) selon la revendication 1 ou la revendication 2.

## Patentansprüche

1. Verbindung, **dadurch gekennzeichnet, dass** sie der folgenden Formel (I) entspricht: 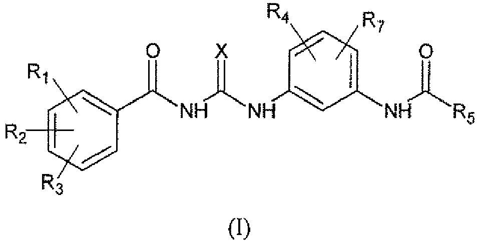 wobei:
- R₁, R₂ und R₃, die identisch oder unterschiedlich und unabhängig voneinander sind, ein Wasserstoff- oder Halogenatom, ein Hydroxylradikal, eine Alkyl-, Perfluoralkyl-, Alcoxy-, Alkylthio- oder Nitrilgruppe darstellen, und vorzugsweise R₁, R₂ und R₃ ein Alcoxyradikal und noch vorzugsweiser ein Methoxyradikal darstellen,
- X O, S oder NH darstellt, und vorzugsweise X NH ist,
- R₄ und R₇, die identisch oder unterschiedlich und unabhängig voneinander sind, ein Wasserstoff- oder Halogenatom oder eine Alkylgruppe darstellen, und vorzugsweise R₄ und R₇ ein Wasserstoff- oder Chloratom, eine Methyl-, Ethyl- oder Isopropylgruppe darstellen,
- R₅ eine der Gruppen darstellt, die ausgewählt sind aus: 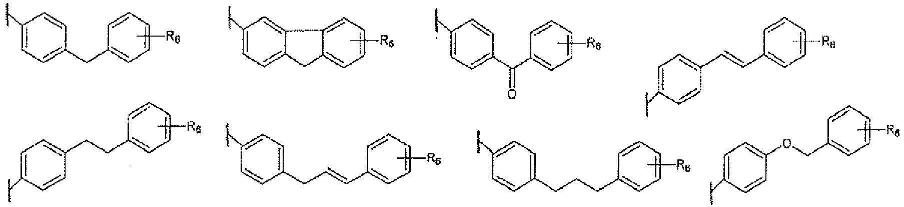 substituiert durch mindestens ein Radikal R₆, das ein Halogenatom oder eine Alkyl-, Alcoxy-, Aminoalkyl-, Thioalkyl- oder Hydroxylgruppe darstellt, und vorzugsweise R₆ ein Halogenatom oder eine Alcoxy- oder Aminoalkylgruppe darstellt.

2. Verbindung der Formel (I) nach Anspruch 1, **dadurch gekennzeichnet, dass** sie ausgewählt ist aus:
- dem Hydrochlorid von *N*-(*N*-(3-(4-benzoylbenzamido)-4-methylphenyl)carbamimidoyl)-3,4,5-trimethoxybenzamid der folgenden Formel: 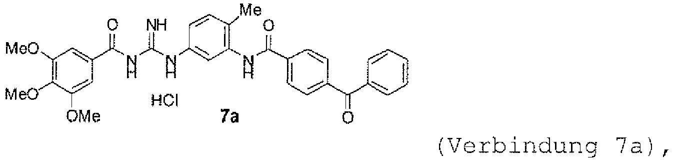
- dem Hydrochlorid von *N*-(*N*-(3-(4-benzoylbenzamido)-4-methylphenyl)carbamimidoyl)-3,4,5-trimethoxybenzamid der folgenden Formel: 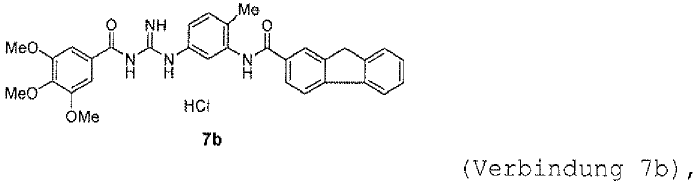
- dem Hydrochlorid von 3,4,5-Trimethoxy-*N*-(*N*-(4-methyl-3-(4-(3-phenylpropyl)benzamido)phenyl)carbamimidoyl)benzamid der folgenden Formel: 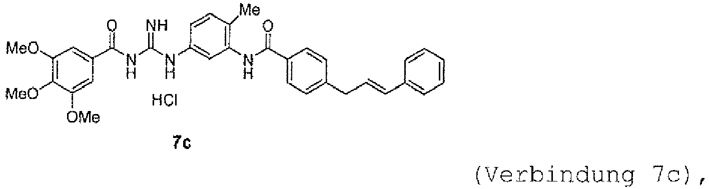
- dem Hydrochlorid von 3,4,5-Trimethoxy-*N*-(*N*-(4-methyl-3(4-phenethylbenzamido)phenyl)carbamimidoyl)benzamid der folgenden Formel: 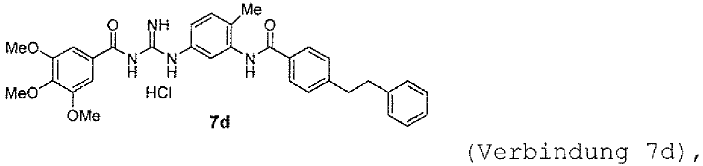
- dem Hydrochlorid von (*E*)-*N*-(*N*-(3-(4-cinnamylbenzamido)-4-methylphenyl)carbamimidoyl)-3,4,5-trimethoxybenzamid der folgenden Formel: 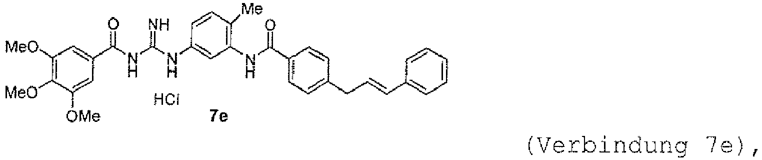
- dem Hydrochlorid von *N*-(*N*-(3-(4-benzylbenzamido)-4-methylphenyl)carbamimidoyl)-3,4,5-trimethoxybenzamid der folgenden Formel: 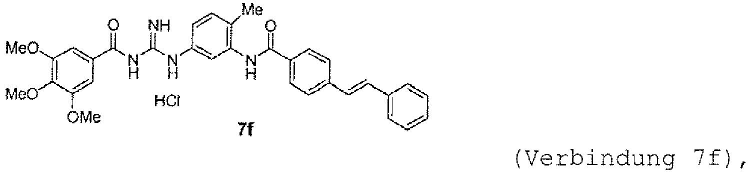
- dem Hydrochlorid von *N*-(*N*-(3-(4-benzylbenzamido)-4-methylphenyl)carbamimidoyl)-3,4,5-trimethoxybenzamid der folgenden Formel: 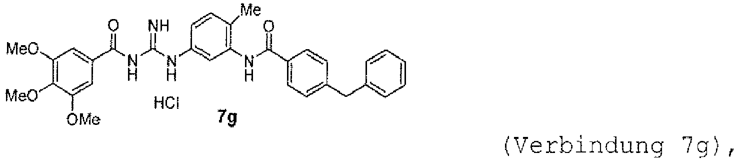
- dem Hydrochlorid von *N*-(*N*-(3-(4-(benzyloxy)benzamido)-4-methylphenyl)carbamimidoyl)-3,4,5-trimethoxybenzamid der folgenden Formel:

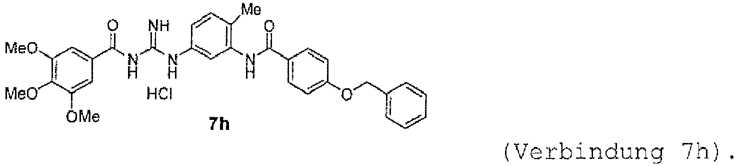

3. Verbindung der Formel (1) nach Anspruch 1 oder Anspruch 2 für ihre Anwendung als Arzneimittel.

4. Verbindung der Formel (1) nach Anspruch 3 für ihre Anwendung als Arzneimittel zur Behandlung von Tumoren, die mit einer Hyperaktivierung des Signalwegs der Hedgehog-Proteine verbunden sind, und vorzugsweise zur Behandlung von Tumoren des Nervengewebes (Medulloblastome, primitive neuroektodermale Tumore, Glioblastome, Meningiome und Oligodendrogliome), von Hauttumoren (basozelluläre Karzinome, Trichoepitheliome), von Tumoren des Muskel- und Knochengewebes (Rhabdomyosarkome, Osteosarkome) oder von Tumoren anderer Gewebe (Nieren, Harnblase, Prostata, Lunge, Magen, Bauchspeicheldrüse).

5. Verbindung der Formel (I) nach Anspruch 3 für ihre Anwendung als Arzneimittel zur Behandlung von Krankheiten des neuro-degenerativen Typs, und vorzugsweise zur Behandlung der Parkinson-Krankheit, von Chorea Huntington, der Alzheimer-Krankheit, der Multiplen Sklerose oder der Motoneuronenerkrankung.

6. Verbindung der Formel (I) nach Anspruch 3 für ihre Anwendung als Arzneimittel zur Behandlung von Krankheiten, die mit der Hirnentwicklung verbunden sind (Holoprosenzephalie), zur Behandlung des Schlaganfalls und von kardiovaskulären Ereignissen sowie von Erkrankungen der Oligodentrozyten und der Schwann-Zellen.

7. Verbindung der Formel (I) nach Anspruch 3 für ihre Anwendung in *vitro* zur Modellierung der Erneuerung der Stammzellen von Mensch oder Tier.

8. Verbindung der Formel (I) nach Anspruch 3 für ihre Anwendung als Arzneimittel zur Behandlung des Diabetes.

9. Pharmazeutische Zusammensetzung, **dadurch gekennzeichnet, dass** sie als Wirkstoff mindestens eine Verbindung der Formel (I) nach Anspruch 1 oder Anspruch 2 und mindestens einen pharmazeutisch akzeptablen Hilfsstoff umfasst.

10. Verwendung einer Verbindung der Formel (I), markiert als Forschungsinstrument, vor allem für die Identifizierung von Molekülen, die imstande sind, mit dem Smoothened-Rezeptor oder einem ähnlichen Rezeptor zu interagieren.

11. Verfahren zur radiologischen Markierung einer Verbindung der Formel (I) nach Anspruch 1 oder Anspruch 2, **dadurch gekennzeichnet, dass** es einen Schritt der Titrierung einer Verbindung der Formel (I) in einer Tritium ³H-Atmosphäre umfasst, und wobei das Verfahren einen vorherigen Halogenierungsschritt umfasst, wenn die Verbindung der Formel (I) von einer Gruppe R₅ substituiert ist, die keine doppelte Ethylenverbindung hat.

12. Verbindung der Formel (I) nach Anspruch 1 oder Anspruch 2, **dadurch gekennzeichnet, dass** mindestens eines ihrer Wasserstoffatome durch ein Tritium ³H-Atom ersetzt wurde und gemäß dem Verfahren von Anspruch 11 herstellbar ist.

13. Screening- und/oder Identifikationsmethode von Liganden der Smo-Verbindungsstellen des Smoothened-Rezeptors, **dadurch gekennzeichnet, dass** es die folgenden Schritte umfasst:
a) Inkontaktversetzen des Smoothened-Rezeptors und mindestens einer Verbindung der Formel (I) für den Erhalt eines Komplexes [Smo-Verbindung der Formel (I)];
b) Inkontaktversetzen des Smoathened-Rezeptors, der Verbindung der Formel (I) und eines zu testenden Moleküls;
c) Ermitteln einer Interaktion zwischen dem Smoothened-Rezeptor und den zu testenden Molekülen durch Vergleich des in Schritt b) rückgewonnenen Smoothened-Rezeptors mit dem Komplex [Smo-Verbindung der Formel (I)]; und
d) Auswählen der zu testenden Moleküle, für die eine Interaktion mit dem Smoothened-Rezeptor gemessen wird.

14. Screening- und/oder Identifikationsmethode nach Anspruch 13, **dadurch gekennzeichnet, dass** der Schritt a) in flüssiger Phase umgesetzt wird, und dass die Methode die folgenden Schritte umfasst:
a) Inkontaktversetzen des Smoothened-Rezeptors und mindestens einer Verbindung der Formel (I) für den Erhalt eines Komplexes [Smo-Verbindung der Formel (I)];
b) Inkontaktversetzen des Smoothened-Rezeptors, der Verbindung der Formel (I) und eines zu testenden Moleküls;
c) Rückgewinnung des eventuell mit einem oder mehreren zu testenden Molekülen und/oder mit der Verbindung der Formel (I) verbundenen Smoothened-Rezeptors;
d) Ermitteln einer Interaktion zwischen dem Smoothened-Rezeptor und den zu testenden Molekülen durch Vergleich des in Schritt c) rückgewonnenen Smoothened-Rezeptors mit dem Komplex [Smo-Verbindung der Formel (I)]; und
e) Auswählen der zu testenden Moleküle, für die eine Interaktion gemessen wird.

15. Identifikationsmethode von Agonisten des Smoothened-Rezeptors, **dadurch gekennzeichnet, dass** sie die Schritte einer der Methoden der Ansprüche 13 oder 14 umfasst, und die folgenden zusätzlichen Schritte:
- Inkontaktversetzen des identifizierten Liganden mit einer Zelle, die infolge der Aktivierung des Smoothened-Rezeptors eine Zellantwort aufweist, und
- Auswählen der Agonistenmoleküle, die imstande sind, die Zellantwort der Zelle zu induzieren.

16. Identifikationsmethode eines antagonistischen Moleküls des Smoothened-Rezeptors, **dadurch gekennzeichnet, dass** sie die folgenden Schritte umfasst:
a) Kultivieren von Zellen, die infolge der Aktivierung des Smoothened-Rezeptors eine Zellantwort aufweisen, mit mindestens einer Verbindung der Formel (I), um die Zellantwort zu induzieren;
b) Inkontaktversetzen der nach Abschluss von Schritt a) erhaltenen Zellen mit einem zu testenden Molekül;
c) Auswählen der Moleküle, die eine Hemmung der Zellantwort der Zellen induzieren.

17. Identifikationsmethode von Zellen wie Tumorzellen, die den Smoothened-Rezeptor exprimieren, **dadurch gekennzeichnet, dass** sie die folgenden Schritte umfasst:
a) Inkontaktversetzen von zu testenden Zellen mit einer markierten Verbindung der Formel (I);
b) Reinigen der Zellen, um die markierte Verbindung der Formel (I) zu entfernen, die sich mit keinem Rezeptor der zu testenden Zellen verbunden hat;
c) Ermitteln der markierten Zellen.

18. Kit, **dadurch gekennzeichnet, dass** es mindestens einen funktionellen Smoothened-Rezeptor und mindestens eine Verbindung der Formel (I) nach Anspruch 1 oder Anspruch 2 umfasst.

## Claims

1. A compound, **characterized in that** it corresponds to the following formula (I): 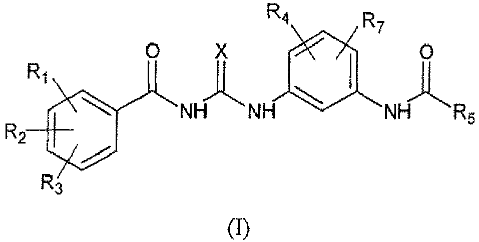 in which:
- R₁, R₂ and R₃, which may be identical or different, independently of one another, represent a hydrogen or halogen atom, a hydroxyl radical, an alkyl, perfluoroalkyl, alkoxy, alkylthio or nitrile group, and preferably R₁, R₂ and R₃ represent an alkoxy radical, and more preferably a methoxy radical,
- X represents O, S or NH, and preferably X is NH,
- R₄ and R₇, which may be identical or different, independently of one another, represent a hydrogen or halogen atom or an alkyl group, and preferably R₄ and R₇ represent a hydrogen or chlorine atom, a methyl, ethyl or isopropyl group,
- R₅ represents one of the groups selected from: 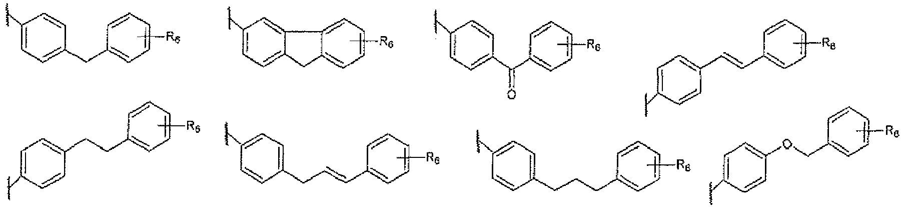 substituted with at least one radical R₆ representing a halogen atom or an alkyl, alkoxy, aminoalkyl, thioalkyl or hydroxyl group, and preferably R₆ represents a halogen atom or an alkoxy or aminoalkyl group.

2. The compound of formula (I) according to claim 1, **characterized in that** it is selected from:
- *N*-(*N*-(3-(4-benzoylbenzamido)-4-methylphenyl)carbamimidoyl)-3,4,5-trimethoxybenzamide hydrochloride of the following formula: 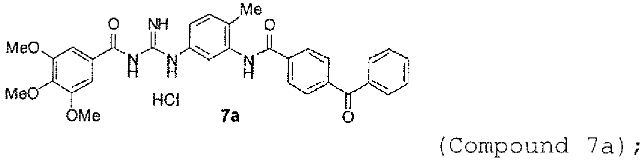
- *N*-(*N*-(3-(4-benzoylbenzamido)-4-methylphenyl)carbamimidoyl)-3,4,5-trimethoxybenzamide hydrochloride of the following formula: 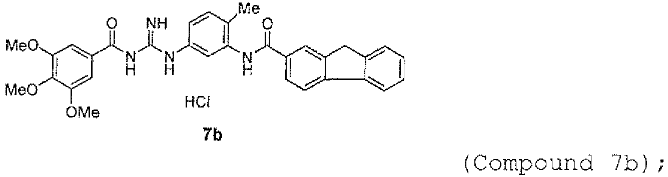
- 3,4,5-trimethoxy-*N*-(*N*-(4-methyl-3-(4-(3-phenylpropyl)benzamido)phenyl)carbamimidoyl)benzamide hydrochloride of the following formula: 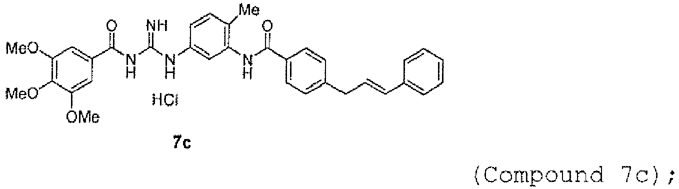
- 3,4,5-trimethoxy-*N*-(*N*-(4-methyl-3-(4-phenethylbenzamido)phenyl)carbamimidoyl)benzamide hydrochloride of the following formula: 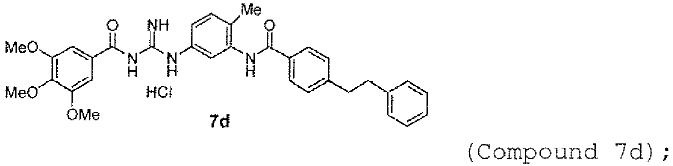
- (*E*)*-N-*(*N-*(3-(4-cinnamylbenzamido)-4-methylphenyl)carbamimidoyl)-3,4,5-trimethoxybenzamide hydrochloride of the following formula: 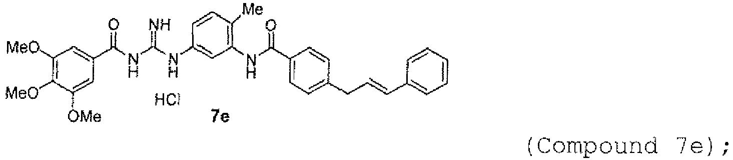
- *N*-(*N*-(3-(4-benzylbenzamido)-4-methylphenyl)carbamimidoyl)-3,4,5-trimethoxybenzamide hydrochloride of the following formula: 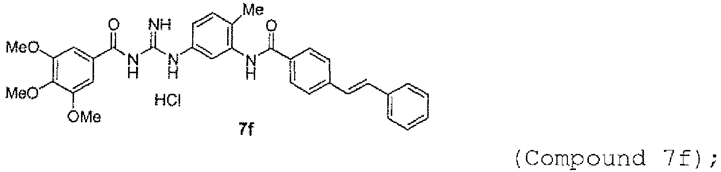
- *N*-(*N*-(3-(4-benzylbenzamido)-4-methylphenyl)carbamimidoyl)-3,4,5-trimethoxybenzamide hydrochloride of the following formula: 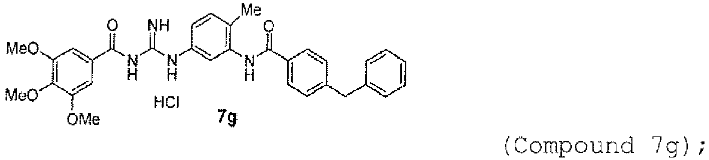
- *N*-(*N*-(3-(4-(benzyloxy)benzamido)-4-methylphenyl)carbamimidoyl)-3,4,5-trimethoxybenzamide hydrochloride of the following formula:

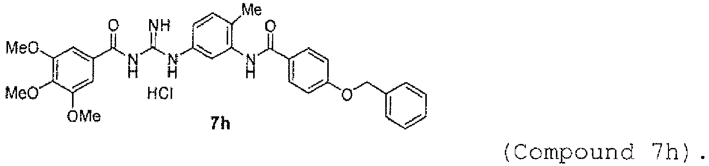

3. The compound of formula (I) according to claim 1 or claim 2, for application as a medicinal product.

4. The compound of formula (I) according to claim 3, for application as a medicinal product for treating tumors associated with hyperactivation of the Hedgehog protein signaling pathway, and preferably for treating nerve tissue tumors (medulloblastomas, neuroectodermal primary tumors, glioblastomas, meningiomas and oligodendrogliomas), skin tumors (basal cell carcinomas, trichoepitheliomas), tumors of muscle and bone tissues (rhabdomyosarcomas, osteosarcomas) or tumors of other tissues (kidney, bladder, prostate, lung, stomach, pancreas).

5. The compound of formula (I) according to claim 3, for application as a medicinal product for treating disorders of the neurodegenerative type, and preferably for treating Parkinson's disease, Huntington's chorea, Alzheimer's disease, multiple sclerosis or motor neuron disease.

6. The compound of formula (I) according to claim 3, for application as a medicinal product for treating diseases connected with cerebral development (holoprosencephaly), for treating cerebrovascular accident and cardiovascular accidents, as well as for diseases of the oligodendrocytes and Schwann cells.

7. The compound of formula (I) according to claim 3, for application *in vitro* for modulating the renewal of human or animal stem cells.

8. The compound of formula (I) according to claim 3, for application as a medicinal product for treating diabetes.

9. A pharmaceutical composition, **characterized in that** it comprises, as active principle, at least one compound of formula (I) according to claim 1 or claim 2 and at least one pharmaceutically acceptable excipient.

10. The use of a labeled compound of formula (I) as a research tool, notably for identifying molecules capable of interacting with the Smoothened receptor or a related receptor.

11. A method of radiolabeling a compound of formula (I) according to claim 1 or claim 2, **characterized in that** it comprises a step of tritiating a compound of formula (I) under an atmosphere of tritium ³H, said method comprising a preliminary step of halogenation when the compound of formula (I) is substituted with a group R₅ without an ethylenic double bond.

12. The compound of formula (I) according to claim 1 or claim 2, **characterized in that** at least one of its hydrogen atoms has been replaced with a tritium atom ³H, and it is obtainable according to the method of claim 11.

13. A method of screening and/or identifying ligands of the Smo binding sites of the Smoothened receptor, **characterized in that** it comprises the following steps:
a) bringing in contact the Smoothened receptor and at least one compound of formula (I) to obtain a complex [Smo-compound of formula (I)];
b) bringing in contact the Smoothened receptor, said compound of formula (I) and a test molecule;
c) detecting interaction between said Smoothened receptor and said test molecules by comparing the Smoothened receptor recovered in step b) with the complex [Smo-compound of formula (I)]; and
d) selecting said test molecules for which interaction with the Smoothened receptor is measured.

14. The method of screening and/or identification according to claim 13, **characterized in that** step a) is carried out in the liquid phase, and **in that** said method comprises the following steps:
a) bringing in contact the Smoothened receptor and at least one compound of formula (I) to obtain a complex [Smo-compound of formula (I)];
b) bringing in contact the Smoothened receptor, said compound of formula (I) and a test molecule;
c) recovering said Smoothened receptor optionally bound to one or more test molecules and/or to said compound of formula (I);
d) detecting interaction between said Smoothened receptor and said test molecules by comparing the Smoothened receptor recovered in step c) with the complex [Smo-compound of formula (I)]; and
e) selecting said test molecules for which interaction is measured.

15. A method of identifying agonists of the Smoothened receptor, **characterized in that** it comprises the steps of one of the methods of claims 13 or 14, and the following additional steps:
- bringing the ligand identified in contact with a cell that displays a cellular response following activation of the Smoothened receptor, and
- selecting the agonist molecules capable of inducing said cellular response of said cell.

16. A method of identifying a molecule that is an antagonist of the Smoothened receptor, **characterized in that** it comprises the following steps:
a) culturing cells that display a cellular response following activation of the Smoothened receptor with at least one compound of formula (I) so as to induce said cellular response;
b) bringing the cells obtained at the end of step a) in contact with a test molecule;
c) selecting the molecules inducing inhibition of said cellular response of said cells.

17. A method of identifying cells, such as tumor cells, expressing the Smoothened receptor, **characterized in that** it comprises the following steps:
a) bringing cells to be tested in contact with a labeled compound of formula (I);
b) cleaning the cells in order to remove said labeled compound of formula (I) that has not bound to any receptor of the cells to be tested;
c) detecting the labeled cells.

18. A kit, **characterized in that** it comprises at least one functional Smoothened receptor and at least one compound of formula (I) according to claim 1 or claim 2.
